(19) Europäisches Patentamt
European Patent Office
Office européen des brevets

(11) Veröffentlichungsnummer : **0 433 822 B1**

(12) # EUROPÄISCHE PATENTSCHRIFT

(45) Veröffentlichungstag der Patentschrift : **08.12.93 Patentblatt 93/49**

(51) Int. Cl.$^5$ : **C07C 69/63,** C07C 69/65, C07C 271/28, C08K 5/10

(21) Anmeldenummer : **90123652.1**

(22) Anmeldetag : **08.12.90**

(54) **Neue Fluorkohlenstoff- und Oxypropylenalkylethergruppen gebunden enthaltende Ester, diese Ester enthaltende Emulsionen und deren Verwendung zur Herstellung von zellhaltigen Kunststoffen nach dem Polyisocyanat-Polyadditionsverfahren.**

(30) Priorität : **19.12.89 DE 3941871**

(43) Veröffentlichungstag der Anmeldung : **26.06.91 Patentblatt 91/26**

(45) Bekanntmachung des Hinweises auf die Patenterteilung : **08.12.93 Patentblatt 93/49**

(84) Benannte Vertragsstaaten : **AT BE CH DE DK ES FR GB IT LI NL SE**

(56) Entgegenhaltungen :
**EP-A- 0 297 822**
**DE-A- 1 593 635**
**DE-A- 2 656 423**
**GB-A- 1 157 320**
**US-A- 3 184 419**

(56) Entgegenhaltungen :
**US-A- 3 398 182**
**PATENT ABSTRACTS OF JAPAN vol. 1, no. 59 (C-651)(77) 08 Juni 1977, & JP-A-52 19751 (ASAHI KASEI) 15 Februar 1977,**
**PATENT ABSTRACTS OF JAPAN vol. 8, no. 255 (C-253)(1692) 21 November 1984, & JP-A-59 135278 (DAINIPPON INK KAGAKU) 03 August 1984,**

(73) Patentinhaber : **BASF Aktiengesellschaft**
**Carl-Bosch-Strasse 38**
**D-67063 Ludwigshafen (DE)**

(72) Erfinder : **Volkert, Otto, Dr.**
**Im Eiertal 10**
**W-6719 Weisenheim (DE)**
Erfinder : **Hinz, Werner, Dr.**
**Hanns-Fay-Strasse 1**
**W-6710 Frankenthal (DE)**

EP 0 433 822 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Erfindungsgegenstände sind neue Fluorkohlenstoff- und Oxypropylenalkylethergruppen gebunden enthaltende Ester der Formeln

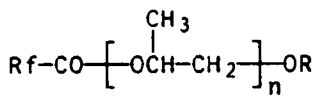

oder

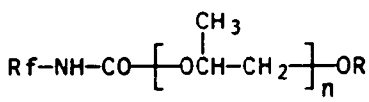

,

lagerstabile, treibmittelhaltige Emulsionen, die enthalten mindestens einen partiell fluorierten oder perfluorierten (cyclo)aliphatischen Kohlenwasserstoff mit 3 bis 8 Kohlenstoffatomen und/oder Schwefelhexafluorid als Treibmittel,
mindestens ein gegebenenfalls modifiziertes organisches Polyisocyanat oder eine höhermolekulare reaktive Wasserstoffverbindung als kohärente Phase und
mindestens einen der vorgenannten Ester als Emulgator
und die Verwendung dieser Emulsionen zur Herstellung von zellhaltigen Kunststoffen nach dem Polyisocyanat-Polyadditionsverfahren.

Oberflächenaktive Substanzen, die als wirksame Gruppen partiell fluorierte und/oder perfluorierte, gegebenenfalls olefinisch ungesättigte organische Rest und Polyoxyalkylengruppen gebunden enthalten, sind bekannt.

Nach Angaben der DE-A-22 44 028 (GB-A-1 371 054) weist ein derartiges oberflächenaktives Mittel folgende drei Bestandteile auf: eine endständige aliphatische Perfluorokohlenstoffgruppe mit mindestens 3 Kohlenstoffatomen, die über eine Etherbrücke mit einer difunktionellen Polyoxyalkylenkette verknüpft ist, die ihrerseits als Endgruppe eine oleophile Gruppe mit einem endständigen Alkylrest mit 3 bis 20 Kohlenstoffatomen besitzt.

Perfluoralkenyl- oder hochfluorierte Alkylreste, die über eine Etherbrücke mit vorzugsweise einer Polyoxyalkyleneinheit verbunden sind, deren zweite Hydroxylgruppe mit einem Alkylrest mit 1 bis 20 Kohlenstoffatomen, einem Alkylphenyl-, einem Alkylnaphthyl-, einem Perfluoralkenyl- oder einem hochfluorierten Alkylrest verethert ist, werden in der DE-A-22 50 718 (GB-A-1 354 138) beschrieben. Nachteilig an diesen Verbindungen ist ihre diffizile Herstellung, insbesondere die schwierige Verknüpfung des perfluorierten oder der perfluorierten Alkylreste mit der Polyoxyalkylengruppe, die überwiegend oder vollständig aus Ethylenoxideinheiten besteht, über eine Etherbrücke.

In dem Referat zu I 5 1058-105, photoempfindliche, flächige Materialien betreffend, werden Perfluorcarbonsäureester mit Polyoxyethylen- und Polyoxyethylen-polyoxypropylen-glykolen beschrieben, die als Endgruppe eine freie, reaktive Hydroxylgruppe aufweisen.

Fluorgruppen enthaltende Ester zur Erhöhung der Öl-, Fett- oder Wasserabstoßung in filmbildenden Polymermaterialien sind bekannt aus der GB-A 1 157 320. Zu ihrer Herstellung werden z.B. Perfluorcarbonsäuren mit mehrwertigen Alkoholen partiell verestert und anschließend die noch verbleibenden freien Hydroxylgruppen acetyliert oder es werden Fluoralkanole mit Polycarbonsäuren verestert.

Die US-A-4 289 892 und US-A-4 356 273 beschreiben reaktive Wasserstoffatome aufweisende Fluorverbindungen mit einer N-Ethylsulfonamidgruppe als Brückenglied und ihre Verwendung als Schaumstabilisator in Polyurethanschaumstoffen. Bei diesen mindestens difunktionellen Fluorverbindungen besteht der Polyoxyalkylenrest aus einem Polyoxyethylen-polyoxypropylenrest mit mittelständigen Oxypropylengruppen und endständigen primären Hydroxylgruppen.

Ähnlich kompliziert aufgebaute perfluoralkylsubstituierte Polyether, bei denen der perfluorierte Alkylrest über eine $-SO_2$-NR-CO-O-Brücke an die Polyetherkette gebunden ist, sind aus der DE-B-22 38 740 (US-A-39 06 027) bekannt. Die nichtionogenen Tenside finden Verwendung als Schaumstabilisatoren, insbesondere für Polyurethanschaumstoffe, als Emulgatoren oder Netzmittel.

Bekannt ist ferner die Verwendung von hoch- oder perfluorierten, niedrigsiedenden Alkanen als Treibmittel für Polyurethanschaumstoffe, da diese alle wichtigen Anforderungen in bezug auf Nichtbrennbarkeit, Toxizität,

Wärmeleitfähigkeit und andere physikalische Eigenschaften erfüllen. (US-A-3 184 419, DE-A- 38 24 354). Problematisch ist allenfalls ihre geringe Löslichkeit in den Aufbaukomponenten zur Herstellung der Polyisocyanat-Polyadditionsprodukte.

Der Mechanismus der Schaumbildung bei der Herstellung von Polyisocyanat-Polyadditionsprodukten und der Einfluß von oberflächenaktiven Hilfsmitteln auf der Grundlage von Siloxan-oxalkylen-copolymeren auf diese Reaktion wurde von B. Kanner et al. (J. of cellular Plastics, January 1969, Seiten 32 bis 39) beschrieben.

Nach Angaben dieser und anderer Publikationen ist als wesentliches Erfordernis für die Bildung von zellhaltigen Polyisocyanat-Polyadditionsprodukten mit gleichförmiger Zellstruktur und guten mechanischen Eigenschaften die homogene Lösung der Treibmittel, wie z.B. des Kohlendioxids und/oder der inerten, niedrigsiedenden Flüssigkeiten, in den organischen Polyisocyanaten und/oder den Verbindungen mit reaktiven Wasserstoffatomen anzusehen ("Blowing Agents for Polyurethanes" von L.M. Zwolinski in Rubber Age, July 1975, Seiten 50 bis 55 und US-A-3 184 419). Falls die Treibmittel nicht in den vorgenannten Aufbaukomponenten löslich sind, werden nur grobporige oder in den meisten Fällen überhaupt keine Schaumstoffe erhalten.

Zur Verminderung dieses Nachteils werden nach Angaben der US-A-4 544 679 spezielle Polyolmischungen mit erhöhter Fluorchlorkohlenwasserstofflöslichkeit eingesetzt und/oder es wird versucht durch Zusatz von teilweise erheblichen Mengen an Lösungsvermittlern homogene Lösungen aus Treibmitteln und den Polyisocyanaten und/oder Polyolen zu erhalten (K. Tanabe, I. Kamemura und S. Kozawa, 28. SPI-Conf. 1984, Seiten 53 bis 57).

Die Aufgabe der vorliegenden Erfindung bestand darin, die als Treibmittel zur Herstellung von zellhaltigen Kunststoffen nach dem Polyisocyanat-Polyadditionsverfahren bekannten und bisher angewandten Fluorchlorkohlenwasserstoffe vollständig oder zumindest teilweise durch andere umweltfreundliche Treibmittel zu ersetzen. Hierfür geeignet erschienen hochoder perfluorierte, niedrigsiedende Kohlenwasserstoffe. Nachteilig an diesen Verbindungen ist jedoch, daß sie in den üblichen Aufbaukomponenten zur Herstellung von Urethan- und/oder Isocyanuratgruppen enthaltenden Kunststoffen schwer löslich oder im wesentlichen unlöslich sind. Nachteilig ist ferner, daß sich die fluorierten Verbindungen, wahrscheinlich aufgrund ihrer sehr niedrigen Oberflächenspannung, in der Regel mittels bekannter Emulgatoren nur sehr schlecht emulgieren lassen, so daß derartige Emulsionen mit Polyhydroxylverbindungen oder organischen Polyisocyanaten als kohärenter Phase eine unzureichende Lagerstabilität aufweisen. Es bedurfte daher zunächst der Entwicklung wirksamer und leicht zugänglicher Emulgatoren und geeigneter lagerbeständiger, treibmittelhaltiger Emulsionen.

Diese Aufgabe konnte überraschenderweise mit neuen Emulgatoren gelöst werden, die bestehen aus einer hydrophoben Fluorkohlenstoffgruppe und einer hydrophilen Oxypropylenalkylethergruppe, die durch ein -CO-O- oder -NH-CO-O- Brückenglied miteinander verknüpft sind.

Gegenstand der Erfindung sind somit Fluorkohlenstoff- und Oxypropylenalkylethergruppen gebunden enthaltende Ester der Formeln

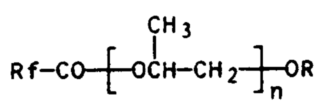

oder

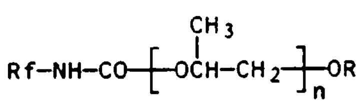

in denen bedeuten:

- Rf eine verzweigte oder vorzugsweise lineare, partiell fluorierte oder perfluorierte Alkylgruppe mit 2 bis 10 Kohlenstoffatomen,
  eine partiell fluorierte oder vorzugsweise perfluorierte Cycloalkylgruppe mit 4 bis 8 Kohlenstoffatomen, vorzugsweise 5 oder 6 Kohlenstoffatomen,
  eine Perfluorphenylgruppe oder
  eine Perfluoralkylphenylgruppe mit 1 bis 6 Kohlenstoffatomen, vorzugsweise 1 bis 3 Kohlenstoffatomen im Perfluoralkylrest,
- R eine verzweigte oder vorzugsweise lineare Alkylgruppe mit 1 bis 4 Kohlenstoffatomen, vorzugsweise 1 bis 2 Kohlenstoffatomen und
- n eine ganze Zahl von 2 bis 70, vorzugsweise 2 bis 50 und insbesondere 5 bis 25.

Ein anderer Gegenstand der Erfindung sind lagerstabile, treibmittelhaltige Emulsionen, die enthalten oder vorzugsweise bestehen aus

i) mindestens einem in (ii) schwer- oder unlöslichen, partiell fluorierten oder perfluorierten, aliphatischen und/oder cycloaliphatischen Kohlenwasserstoff mit 3 bis 8 Kohlenstoffatomen und/oder Schwefelhexafluorid,

ii) mindestens einem organischen und/oder modifizierten organischen Polyisocyanat oder mindestens einer höhermolekulare Verbindung mit mindestens zwei reaktiven Wasserstoffatomen oder einer Mischung aus mindestens einer höhermolekularen Verbindung mit mindestens zwei reaktiven Wasserstoffatomen und mindestens einem niedermolekularen Kettenverlängerungs- und/oder Vernetzungsmittel und

iii) mindestens einem Ester der Formeln

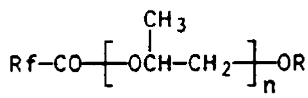

oder

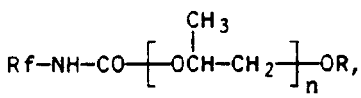

in denen Rf, R und n die vorgenannte Bedeutung haben, und die Verwendung dieser lagerstabilen, treibmittelhaltigen Emulsionen zur Herstellung von zellhaltigen Kunststoffen nach dem Polyisocyanat-Polyadditionsverfahren, vorzugsweise zur Herstellung von Polyurethan-Hartschaumstoffen.

Die neuen, als Emulgatoren geeigneten Fluorkohlenstoff- und Oxypropylenalkylethergruppen gebunden enthaltende Carbonsäure- oder Carbamidsäureester sind durch Umsetzung von partiell fluorierten oder perfluorierten aliphatischen oder aromatischen Carbonsäuren oder Isocyanaten mit Polyoxypropylenglykolmonoalkylethern leicht zugänglich, weisen im Vergleich zu den ebenfalls geeigenten, jedoch nur schwierig herstellbaren Kammpolymeren, bestehend aus einer Polyacrylathauptkette und Seitengruppen aus einem fluorierten organischen Rest und einem Polyoxyethylen- oder Polyoxypropylenrest, eine relativ niedere Viskosität auf, die ihre Dosier- und Verarbeitbarkeit erleichtert und bilden Emulsionen, die auch mit einem hohen Gehalt, z.B. von mehr als 40 Gew.-%, an hochfluorierten oder perfluorierten Treibmitteln in Polyhydroxylverbindungen, vorzugsweise Polyether-polyolen, über mehrere Wochen lagerstabil sind und in diesem Zeitraum problemlos auf üblichen Schäumanlagen verarbeitet werden können.

Vorteilhaft ist ferner, daß die Emulgatorwirkung der neuen Fluorkohlenstoff-und Oxypropylenalkylethergruppen gebunden enthaltenden Ester überraschenderweise durch die zusätzliche Mitverwendung spezieller Schaumstabilisatoren auf Silikonbasis verstärkt und dadurch die Güte der Emulsion verbessert werden kann.

Durch die gute Emulgierung der in Betracht kommenden schwer- oder unlöslichen, partiell fluorierten oder perflourierten, aliphatischen und/oder cycloaliphatischen Kohlenwasserstoffe und/oder Schwefelhexafluorid als Treibmittel und ihre Verdampfung durch die bei der Polyisocyanat-Polyadditionsreaktion entstehende Wärme werden überraschenderweise zellige Kunststoffe mit gleichmäßiger feinzelliger Zellstruktur erhalten.

Zu den neuen Fluorkohlenstoff- und Oxypropylenalkylethergruppen gebunden enthaltenden Estern, ihre Herstellung, den erfindungsgemäß zur Emulsionsbildung geeigneten Treibmitteln und den Aufbaukomponenten zur Herstellung der zelligen Polyisocyanat-Polyadditionsprodukte, vorzugsweise der Urethangruppen oder Urethan- und Isocyanuratgruppen enthaltenden Hartschaumstoffe, ist im einzelnen folgendes auszuführen.

Die neuen Fluorkohlenstoff- und Oxypropylenalkylethergruppen gebunden enthaltende Ester besitzen, wie bereits dargelegt wurde, die Struktur

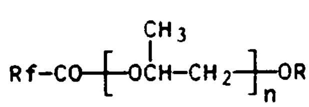

oder

$$Rf{-}NH{-}CO{-}\left[{-}O\overset{CH_3}{\overset{|}{C}}H{-}CH_2{-}\right]_n{-}OR \quad ,$$

wobei bedeuten:

Rf eine verzweigte oder vorzugsweise lineare, partiell fluorierte Alkylgruppe mit 2 bis 10, vorzugsweise 3 bis 6 Kohlenstoffatomen, die insbesondere durch die Formel $C_xF_{2x+1}$-$(CH_2)_y$- beschrieben wird, in der x eine Zahl von 1 bis 9, y eine Zahl von 1 bis 2 und die Summe aus x und y maximal 10 sind,
eine verzweigte oder vorzugsweise lineare, perfluorierte Alkylgruppe mit 2 bis 10, vorzugsweise 2 bis 8 Kohlenstoffatomen, die insbesondere durch die Formel $C_xF_{2x+1}$ beschrieben wird, in der x eine Zahl von 2 bis 10 ist,
eine partiell fluorierte oder vorzugsweise eine perfluorierte Cycloalkylgruppe mit 4 bis 8, vorzugsweise 5 bis 6 Kohlenstoffatomen, wie z.B. eine perfluorierte Cyclobutyl-, Cycloheptyl-, Cyclooctylgruppe und vorzugsweise eine perfluorierte Cyclopentyl- oder Cyclohexylgruppe,
eine perfluorierte Phenylgruppe oder
eine Perfluoralkylphenylgruppe mit 1 bis 6, vorzugsweise 1 bis 3 und insbesondere einem Kohlenstoffatom in der Perfluoralkylgruppe, wie z.B. eine Perfluorhexyl-, Perfluorbutyl-, Perfluor-sek-butyl-, Perfluorpropyl-, Perfluorisopropyl-, Perfluorethyl- und insbesondere eine Perfluormethylphenylgruppe,

R eine verzweigte oder vorzugsweise lineare Alkylgruppe mit 1 bis 4, vorzugsweise 1 bis 2 Kohlenstoffatomen, wie z.B. eine n-Butyl-, sek-Butyl-, n- und iso-Propyl-, vorzugweise Ethyl- und insbesondere Methylgruppe und

n eine ganze Zahl von 2 bis 70, vorzugsweise 2 bis 50 und insbesondere 5 bis 25.

Zur Emulgierung können die Fluorkohlenstoff- und Oxypropylenalkylethergruppen gebunden enthaltenden Carbonsäure- oder Carbamidsäureester einzeln oder in Form einer Mischung aus mindestens 2 Emulgatoren eingesetzt werden.

Sofern die Gruppe Rf nur partiell fluoriert ist, sollte diese zweckmäßigerweise einen Fluorgehalt von mindestens 40 Gew.-%, vorzugsweise von mindestens 70 Gew.-%, bezogen auf das Gewicht von Rf besitzen.

Die erfindungsgemäßen Fluorkohlenstoff-Oxypropylenalkylether-Ester können beispielsweise dargestellt werden durch Veresterung von perfluorierten oder teilfluorierten aliphatischen Carbonsäuren, perfluorierten aromatischen Carbonsäuren oder mit Perfluoralkylgruppen substituierten aromatischen Carbonsäuren oder den entsprechenden Carbonsäurederivaten, vorzugsweise Carbonsäurechloriden, mit Polyoxypropylenglykolmonoalkylethern mit Molekulargewichten von 148 bis 4134, vorzugsweise von 322 bis 1524, die ihrerseits erhalten werden durch anionische Polymerisation von 1.2-Propylenoxid an ein lineares oder verzweigtes Alkanol mit 1 bis 4 Kohlenstoffatomen, z.B. n-Butanol, n- oder iso-Propanol, vorzugsweise Ethanol oder insbesondere Methanol. Nach einer anderen Verfahrensvariante können als teilfluorierte oder perfluorierte Carbonsäurederivate die entsprechenden Alkylester mit 1 bis 3 Kohlenstoffatomen oder Hydroxyalkylester mit 2 bis 4 Kohlenstoffatomen, z.B. die entsprechenden Methyl-, Ethyl-, Isopropyl-oder 2-Hydroxylethylester mit den vorgenannten Polyoxypropylenglykolmonoalkylether umgeestert werden. Besonders vorteilhaft verläuft die Addition der Polyoxypropylenglykolmonoalkylethern an teilfluorierte oder perfluorierte, aliphatische oder aromatische Isocyanate, so daß dieses Verfahren zur Herstellung der Fluorkohlenstoff- und Oxypropylenalkylethergruppen gebunden enthaltenden Carbamidsäureester bevorzugt ist.

Als Aufbaukomponenten besonders bewährt haben sich und daher vorzugsweise Anwendung finden Perfluorbuttersäure, insbesondere Perfluorbuttersäurechlorid, Perfluorhexylessigsäure insbesondere Perfluorhexyl-acetylchlorid, Perfluorbenzoesäure, insbesondere Perfluorbenzoylchlorid und 3-Trifluormethyl-phenylisocyanat, so daß die Gruppe Rf besteht aus einer der Gruppen $C_3F_7$-, $C_6F_{13}$-$CH_2$-, $C_6F_5$- oder $CF_3$-$C_6H_4$- und als Polyoxypropylenglykolmonoalkylether, Polyoxypropylenglykolmonomethylether mit einem Molekulargewicht von 380 bis 1366.

Als Emulgatoren kommen somit bevorzugt zur Anwendung und werden hergestellt nach einem der vorgenannten Verfahren Ester oder Carbamidsäureester der Formeln

$$C_3F_7{-}CO{-}\left[{-}O\overset{CH_3}{\overset{|}{C}}H{-}CH_2{-}\right]_m{-}OCH_3 \quad ,$$

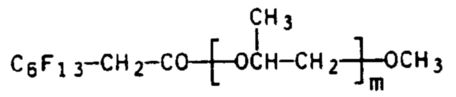

,

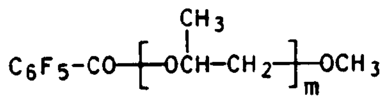

und

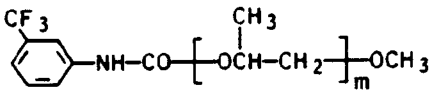

,

in denen m einen Wert von 6 bis 23 besitzt.

Die erfindungsgemäßen Fluorkohlenstoff- und Oxypropylenalkylethergruppen gebunden enthaltenden Carbonsäure- oder Carbamidsäureester finden Verwendung zur Herstellung von lagerstabilen, treibmittelhaltigen Emulsionen, die wiederum zur Herstellung von zellhaltigen Kunststoffen, vorzugsweise Urethan- oder Urethan- und Isocyanuratgruppen enthaltenden Hartschaumstoffen, nach dem Polyisocyanat-Polyadditionsverfahren vorzüglich geeignet sind.

Derartige zellhaltige Kunststoffe werden hergestellt nach dem Polyisocyanat-Polyadditionsverfahren durch Umsetzung von

a) organischen und/oder modifizierten organischen Polyisocyanaten mit

b) mindestens einer höhermolekularen Verbindung mit mindestens zwei reaktiven Wasserstoffatomen und gegebenenfalls

c) niedermolekularen Kettenverlängerungs- und/oder Vernetzungsmittel

in Gegenwart von

d) Treibmitteln,

e) Katalysatoren,

f) Hilfsmitteln und/oder Zusatzstoffen.

Als Aufbaukomponente (a) bis (f) werden zweckmäßigerweise die nachstehend beschriebenen Verbindungen verwendet, zu denen im einzelnen folgendes auszuführen ist:

a) Als organische Polyisocyanate kommen die an sich bekannten aliphatischen$_1$ cycloaliphatischen, araliphatischen und vorzugsweise aromatischen mehrwertigen Isocyanate in Frage.

Im einzelnen seien beispielhaft genannt: Alkylen-diisocyanate mit 4 bis 12 Kohlenstoffatomen im Alkylenrest, wie 1,12-Dodecan-diisocyanat, 2-Ethyl-tetramethylen-diisocyanat-1,4, 2-Methyl-pentamethylen-diisocyanat-1,5, Tetramethylen-diisocyanat-1,4 und vorzugsweise Hexamethylen-diisocyanat-1,6; cycloaliphatische Diisocyanate, wie Cyclohexan-1,3-und -1,4-diisocyanat sowie beliebige Gemische dieser Isomeren, 1-Isocyanato-3,3,5-trimethyl-5-isocyanatomethyl-cyclohexan (Isophoron-diisocyanat), 2,4- und 2,6-Hexahydrotoluylen-diisocyanat sowie die entsprechenden Isomerengemische, 4,4'-, 2,2'- und 2,4'-Dicyclohexylmethan-diisocyanat sowie die entsprechenden Isomerengemische und vorzugsweise aromatischen Di- und Polyisocyanate, wie z.B. 2,4-und 2,6-Toluylen-diisocyanat und die entsprechenden Isomerengemische, 4,4'-, 2,4'- und 2,2'-Diphenylmethan-diisocyanat und die entsprechenden Isomerengemische, Mischungen aus 4,4'- und 2,4'-Diphenylmethan-diisocyanaten, Polyphenyl-polymethylen-polyisocyanate, Mischungen aus 4,4'-, 2,4'- und 2,2'-Diphenylmethan-diisocyanaten und Polyphenylpolymethylen-polyisocyanaten (Roh-MDI) und Mischungen aus Roh-MDI und Toluylen-diisocyanaten. Die organischen Di- und Polyisocyanate können einzeln oder in Form von Mischungen eingesetzt werden.

Häufig werden auch sogenannte modifizierte mehrwertige Isocyanate, d.h. Produkte, die durch chemische Umsetzung organischer Di- und/oder Polyisocyanate erhalten werden, verwendet. Beispielhaft genannt seien Ester-, Harnstoff-, Biuret-, Allophanat-, Carbodiimid-, Isocyanurat-, Uretdion- und/oder Urethangruppen enthaltende Di- und/oder Polyisocyanate. Im einzelnen kommen beispielsweise in Betracht: Urethangruppen enthaltende organische, vorzugsweise aromatische Polyisocyanate mit NCO-Gehalten von 33,6 bis 15 Gew.%, vorzugsweise von 31 bis 21 Gew.%, bezogen auf das Gesamtgewicht, beispielsweise mit niedermolekularen Diolen, Triolen, Dialkylenglykolen, Trialkylenglykolen oder Polyoxyalkylenglykolen mit Molekulargewichten bis 800 modifiziertes 4,4'-Diphenylmethan-diisocyanat oder 2,4- bzw. 2,6-Toluylen-diisocyanat, wobei als Di- bzw. Polyoxyalkylen-glykole, die einzeln oder als Gemische eingesetzt werden können, beispielsweise genannt seien: Diethylen-, Dipropylen-, Polyoxyethylen-, Polyoxypropylen- und Polyoxypropylen-polyoxyethylen-glykole. Geeignet sind auch NCO-Gruppen enthaltende Prepolymere mit NCO-Gehalten von 25 bis 3,5 Gew.%, vorzugsweise von 21 bis 14 Gew.%, bezogen auf das Gesamtgewicht, hergestellt aus den nachfolgend beschriebenen Polyester- und/oder vorzugsweise Polyether-polyolen und 4,4'-Diphenylmethan-diisocyanat, Mischungen aus 2,4'- und 4,4'-Diphenylmethan-diisocyanat, 2,4- und/oder 2,6-Toluylen-diisocyanaten oder Roh-MDI. Bewährt haben sich ferner flüssige Carbodiimidgruppen und/oder Isocyanuratringe enthaltende Polyisocyanate mit NCO-Gehalten von 33,6 bis 15, vorzugsweise 31 bis 21 Gew.%, bezogen auf das Gesamtgewicht, z.B. auf Basis von 4,4'-, 2,4'- und/oder 2,2'-Diphenylmethan-diisocyanat und/oder 2,4- und/oder 2,6-Toluylen-diisocyanat.

Die modifizierten Polyisocyanate können miteinander oder mit unmodifizierten organischen Polyisocyanaten wie z.B. 2,4'-, 4,4'-Diphenylmethan-diisocyanat, Roh-MDI, 2,4- und/oder 2,6-Toluylendiisocyanat gegebenenfalls gemischt werden.

Besonders bewährt haben sich als organische Polyisocyanate und kommen vorzugsweise zur Anwendung zur Herstellung von zelligen Elastomeren: NCO-Gruppen enthaltende Prepolymere mit einem NCO-Gehalt von 25 bis 9 Gew.%, insbesondere auf Basis von Polyether- oder Polyester-polyolen und einem oder mehreren Diphenylmethan-diisocyanat-Isomeren, vorteilhafterweise 4,4'-Diphenylmethan-diisocyanat und/oder modifizierte Urethangruppen enthaltende organische Polyisocyanate mit einem NCO-Gehalt von 33,6 bis 15 Gew.%, insbesondere auf Basis von 4,4'-Diphenylmethan-diisocyanat oder Diphenylmethan-diisocyanat-Isomerengemischen, zur Herstellung von flexiblen Polyurethanschaumstoffen: Mischungen aus 2,4- und 2,6-Toluylen-diisocyanaten, Mischungen aus Toluylen-diisocyanaten und Roh-MDI oder insbesondere Mischungen aus den vorgenannten Prepolymeren auf Basis von Diphenylmethan-diisocyanat-Isomeren und Roh-MDI und zur Herstellung von Polyurethan- oder Polyurethan-Polyisocyanurat-Hartschaumstoffen: Roh-MDI.

b) Als höhermolekulare Verbindungen b) mit mindestens zwei reaktiven Wasserstoffatomen werden zweckmäßigerweise solche mit einer Funktionalität von 2 bis 8, vorzugsweise 2 bis 6 und einem Molekulargewicht von 380 bis 8000, vorzugsweise von 1200 bis 6000 verwendet. Bewährt haben sich beispielsweise Polyole ausgewählt aus der Gruppe der Polyether-polyole, Polyester-polyole, Polythioether-polyole, Polyester-amide, hydroxylgruppenhaltigen Polyacetale und hydroxylgruppenhaltigen aliphatischen Polycarbonate oder Mischungen aus mindestens zwei der genannten Polyole. Vorzugsweise Anwendung finden Polyester-polyole und/oder Polyether-polyole.

Geeignete Polyester-polyole können beispielsweise aus organischen Dicarbonsäuren mit 2 bis 12 Kohlenstoffatomen, vorzugsweise aliphatischen Dicarbonsäuren mit 4 bis 6 Kohlenstoffatomen und mehrwertigen Alkoholen, vorzugsweise Diolen, mit 2 bis 12 Kohlenstoffatomen, vorzugsweise 2 bis 6 Kohlenstoffatomen hergestellt werden. Als Dicarbonsäuren kommen beispielsweise in Betracht: Bernsteinsäure, Glutarsäure, Adipinsäure, Korksäure, Azelainsäure, Sebacinsäure, Decandicarbonsäure, Maleinsäure, Fumarsäure, Phthalsäure, Isophthalsäure und Terephthalsäure. Die Dicarbonsäuren können dabei sowohl einzeln als auch im Gemisch untereinander verwendet werden. Anstelle der freien Dicarbonsäuren können auch die entsprechenden Dicarbonsäurederivate, wie z.B. Dicarbonsäuremono- und/oder -diester von Alkoholen mit 1 bis 4 Kohlenstoffatomen oder Dicarbonsäureanhydride eingesetzt werden. Vorzugsweise verwendet werden Dicarbonsäuregemische aus Bernstein-, Glutar- und Adipinsäure in Mengenverhältnissen von beispielsweise 20 bis 35 : 35 bis 50 : 20 bis 32 Gew.-Teilen, und insbesondere Adipinsäure. Beispiele für zwei- und mehrwertige Alkohole, insbesondere Diole sind: Ethandiol, Diethylenglykol, 1,2- bzw. 1,3-Propandiol, Dipropylenglykol, 1,4-Butandiol, 1,5-Pentandiol, 1,6-Hexandiol, 1,10-Decandiol, Glycerin und Trimethylolpropan. Vorzugsweise verwendet werden Ethandiol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol. Eingesetzt werden können ferner Polyester-polyole aus Lactonen, z.B. ε-Caprolacton oder Hydroxycarbonsäuren, z.B. ω-Hydroxycapronsäure.

Zur Herstellung der Polyester-polyole können die organischen, z.B. aromatischen und vorzugsweise aliphatischen Polycarbonsäuren und/oder -derivate und mehrwertigen Alkohole katalysatorfrei oder vorzugsweise in Gegenwart von Veresterungskatalysatoren, zweckmäßigerweise in einer Atmosphäre aus Inertgasen, wie z.B. Stickstoff, Kohlenmonoxid, Helium, Argon u.a. in der Schmelze bei Temperaturen von 150 bis 250°C, vorzugsweise 180 bis 220°C, gegebenenfalls unter vermindertem Druck bis zu der gewünschten Säurezahl, die vorteilhafterweise kleiner als 10, vorzugsweise kleiner als 2 ist, polykondensiert werden. Nach einer bevorzugten Ausführungsform wird das Veresterungsgemisch bei den obengenannten Temperaturen bis zu einer Säurezahl von 80 bis 30, vorzugsweise 40 bis 30, unter Normaldruck und anschließend unter einem Druck von kleiner als 500 mbar, vorzugsweise 50 bis 150 mbar, polykondensiert.

Als Veresterungskatalysatoren kommen beispielsweise Eisen-, Cadmium-, Kobalt-, Blei-, Zink-, Antimon-, Magnesium-, Titan- und Zinnkatalysatoren in Form von Metallen, Metalloxiden oder Metallsalzen in Betracht. Die Polykondensation kann jedoch auch in flüssiger Phase in Gegenwart von Verdünnungs- und/oder Schleppmitteln, wie z.B. Benzol, Toluol, Xylol oder Chlorbenzol, zur azeotropen Abdestillation des Kondensationswassers durchgeführt werden.

Zur Herstellung der Polyester-polyole werden die organischen Polycarbonsäuren und/oder -derivate und mehrwertigen Alkohole vorteilhafterweise im Molverhältnis von 1:1 bis 1,8, vorzugsweise 1:1,05 bis 1,2 polykondensiert.

Die erhaltenen Polyester-polyole besitzen vorzugsweise eine Funktionalität von 2 bis 4, insbesondere 2 bis 3 und ein Molekulargewicht von 480 bis 3000, vorzugsweise 1200 bis 3000 und insbesondere 1800 bis 2500.

Insbesondere als Polyole verwendet werden jedoch Polyether-polyole, die nach bekannten Verfahren, beispielsweise durch anionische Polymerisation mit Alkalihydroxiden, wie Natrium- oder Kaliumhydroxid oder Alkalialkoholaten, wie Natriummethylat, Natrium- oder Kaliumethylat oder Kaliumisopropylat als Katalysatoren und unter Zusatz mindestens eines Startermoleküls das 2 bis 8, vorzugsweise 2 bis 6 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 4,4'-, 2,4'- und 2,2'-Diamino-diphenylmethan.

Als Startermoleküle kommen ferner in Betracht: Alkanolamine, Dialkanolamine und/oder Trialkanolamine, wie Ethanolamin, Diethanolamin, N-Methyl- und N-Ethyl-ethanolamin, N-Methyl- und N-Ethyl-diethanolamin und Triethanolamin und Ammoniak. Vorzugsweise verwendet werden mehrwertige, insbesondere zwei- und/oder dreiwertige Alkohole, wie Ethandiol, Propandiol-1,2 und -1,3, Diethylenglykol, Dipropylenglykol, Butandiol-1,4, Hexandiol-1,6, Glycerin, Trimethylol-propan, Pentaerythrit, Sorbit und Saccharose.

Die Polyether-polyole, vorzugsweise Polyoxypropylen- und Polyoxypropylen-polyoxyethylen-polyole, besitzen eine Funktionalität von vorzugsweise 2 bis 6 und insbesondere 2 bis 4 und Molekulargewichte von 380 bis 8000, vorzugsweise 400 bis 6000 und insbesondere 400 bis 1800 und geeignete Polyoxytetramethylen-glykole ein Molekulargewicht bis ungefähr 3500.

Als Polyether-polyole eignen sich ferner polymermodifizierte Polyether-polyole, vorzugsweise Pfropf-polyether-polyole, insbesondere solche auf Styrol- und/oder Acrylnitrilbasis, die durch in situ Polymerisation von Acrylnitril, Styrol oder vorzugsweise Mischungen aus Styrol und Acrylnitril, z.B. im Gewichtsverhältnis 90 : 10 bis 10 : 90, vorzugsweise 70 : 30 bis 30 : 70, in zweckmäßigerweise den vorgenannten Polyether-polyolen analog den Angaben der deutschen Patentschriften 11 11 394, 12 22 669 (US 3 304 273, 3 383 351, 3 523 093), 11 52 536 (GB 10 40 452) und 11 52 537 (GB 987 618) hergestellt werden, sowie Polyether-polyoldispersionen, die als disperse Phase, üblicherweise in einer Menge von 1 bis 50 Gew.%, vorzugsweise 2 bis 25 Gew.%, enthalten: z.B. Polyharnstoffe, Polyhydrazide, tert.-Aminogruppen gebunden enthaltende Polyurethane und/oder Melamin und die z.B. beschrieben werden in der EP-B-011 752 (US 4 304 708), US-A-4 374 209 und DE-A-32 31 497.

Die Polyether-polyole können ebenso wie die Polyester-polyole einzeln oder in Form von Mischungen verwendet werden. Ferner können sie mit den Pfropf-polyether-polyolen oder Polyester-polyolen sowie den hydroxylgruppenhaltigen Polyesteramiden, Polyacetalen und/oder Polycarbonaten gemischt werden.

Als hydroxylgruppenhaltige Polyacetale kommen z.B. die aus Glykolen, wie Diethylenglykol, Triethylenglykol, 4,4' -Dihydroxyethoxy-diphenyl-dimethylmethan, Hexandiol und Formaldehyd herstellbaren Verbindungen in Frage. Auch durch Polymerisation cyclischer Acetale lassen sich geeignete Polyacetale herstellen.

Als Hydroxylgruppen aufweisende Polycarbonate kommen solche der an sich bekannten Art in Betracht, die beispielsweise durch Umsetzung von Diolen, wie Propandiol-(l,3), Butandiol-(1,4) und/oder Hexandiol-(1,6), Diethylenglykol, Triethylenglykol oder Tetraethylenglykol mit Diarylcarbonaten, z.B. Diphenylcarbonat, oder Phosgen hergestellt werden können.

Zu den Polyesteramiden zählen z.B. die aus mehrwertigen, gesättigten und/oder ungesättigten Carbonsäuren bzw. deren Anhydriden und mehrwertigen gesättigten und/oder ungesättigten Aminoalkoholen oder Mischungen aus mehrwertigen Alkoholen und Aminoalkoholen und/oder Polyaminen gewonnenen, vorwiegend linearen Kondensate.

c) Die Polyisocyanat-Polyadditionsprodukte und vorzugsweise Urethan- oder Urethan- und Isocyanuratgruppen enthaltende Schaumstoffe können ohne oder unter Mitverwendung von Kettenverlängerungs- und/oder Vernetzungsmitteln hergestellt werden. Zur Modifizierung der mechanischen Eigenschaften, z.B. der Härte, kann sich jedoch der Zusatz von Kettenverlängerungsmitteln, vernetzungsmitteln oder gegebenenfalls auch Gemischen davon als vorteilhaft erweisen. Als Kettenverlängerungs- und/oder Vernetzungsmittel verwendet werden Diole und/oder Triole mit Molekulargewichten kleiner als 400, vorzugsweise von 60 bis 300. In Betracht kommen beispielsweise aliphatische, cycloaliphatische und/oder araliphatische Diole mit 2 bis 14, vorzugsweise 4 bis 10 Kohlenstoffatomen, wie z.B. Ethylenglykol, Propandiol-1,3, Decandiol-1,10, o-, m-, p-Dihydroxycyclohexan, Diethylenglykol, Dipropylenglykol und vorzugsweise Butandiol-1,4, Hexandiol-1,6 und Bis-(2-hydroxyethyl)-hydrochinon, Triole, wie 1,2,4-, 1,3,5-Trihydroxycyclohexan, Glycerin und Trimethylolpropan und niedermolekulare hydroxylgruppenhaltige Polyalkylenoxide auf Basis Ethylen- und/oder 1,2-Propylenoxid und den vorgenannten Diolen und/oder Triolen als Startermolekülen.

Die Kettenverlängerungsmittel und/oder vernetzungsmittel (c) können einzeln oder als Mischungen verwendet werden.

Sofern Kettenverlängerungsmittel, Vernetzungsmittel oder Mischungen davon Anwendung finden, kommen diese zweckmäßigerweise in Mengen von 2 bis 60 Gew.%, vorzugsweise 8 bis 50 Gew.% und insbesondere 10 bis 40 Gew.%, bezogen auf das Gewicht der Komponenten (b) und (c) zum Einsatz.

d) Als Treibmittel (d) bzw. (i) werden vorteilhafterweise niedrigsiedende, in (a), (b), (c) oder Mischungen aus (b) und (c) schwer- oder unlösliche, fluorierte Verbindungen verwendet, die ausgewählt sind aus der Gruppe der partiell fluorierten oder perfluorierten Kohlenwasserstoffe sowie Schwefelhexafluorid. Geeignet sind insbesondere teilweise oder vollständig fluorierte, bei Raumtemperatur gasförmige oder flüssige, aliphatische oder cycloaliphatische Kohlenwasserstoffe mit 3 bis 8 C-Atomen, vorzugsweise mit 3 bis 6 C-Atomen, wobei die gasförmigen Fluoralkane unter erhöhtem Druck, beispielsweise unter einem Druck bis 100 bar, vorzugsweise von 1 bis 50 bar und insbesondere von 2 bis 10 bar verflüssigt und in flüssiger Form emulgiert werden. Als bei Raumtemperatur gasförmige, aliphatische oder cycloaliphatische Perfluoralkane seien z.B. genannt: Perfluorpropan, Perfluorbutan und Perfluorcyclobutan. Geeignete bei Raumtemperatur flüssige, aliphatische oder cycloaliphatische Perfluoralkane sind z.B. Perfluorpentan, Perfluorhexan, Perfluorheptan und Perfluoroctan oder Perfluorcyclopentan und Perfluorcyclohexan. Als partiell fluorierte Alkane kommen zweckmäßigerweise in Betracht: Hexafluorpropan und/oder Heptafluorpropan. Besonders bewährt haben sich und daher vorzugsweise Anwendung finden Heptafluorpropan, Perfluorcyclobutan, Perfluorpentan und Perfluorhexan. Die beispielhaft genannten partiell fluorierten oder perfluorierten Kohlenwasserstoffe sowie das Schwefelhexafluorid können einzeln oder in Form von Mischungen aus zwei oder mehreren Treibmitteln eingesetzt werden.

Die als Emulgator geeigneten Fluorkohlenstoff- und Oxypropylenalkylethergruppen gebunden enthaltenden Carbonsäure und/oder Carbamidsäureester werden zweckmäßigerweise in einer Menge von 0,01 bis 6 Gew.-Teilen, vorzugsweise 0,1 bis 3,5 Gew.-Teilen und insbesondere 0,5 bis 2,0 Gew.-Teilen, bezogen auf 100 Gew.-Teile der Aufbaukomponenten (a) oder (b) oder der Mischung aus (b) und (c) eingesetzt.

Zur Emulgierung der als Treibmittel (d) verwendbaren partiell fluorierten Kohlenwasserstoffe, perfluorierten Kohlenwasserstoffe und/oder Schwefelhexafluorid mittels der oben beschriebenen neuen Carbon- oder Carbamidsäureester eignen sich die organischen und/oder modifizierten organischen Polyisocyanate (a) und die höhermolekularen Verbindungen mit mindestens zwei reaktiven Wasserstoffatomen (b). Geeignet sind ferner Mischungen aus (b) und niedermolekularen Kettenverlängerungs- und/oder -vernetzungsmitteln (c). Die Treibmittel (d), die vorteilhafterweise in einer Menge von i bis 150 Gew.-Teilen, vorzugsweise 1 bis 70 Gew.-Teilen und insbesondere 5 bis 50 Gew.-Teilen pro 100 Gew.-Teilen von (a) oder (b) Anwendung finden, können zur Bildung der lagerstabilen Emulsionen sowohl in (a) wie in (b) oder in Mischungen aus (b) und (c) emulgiert werden. Aus verarbeitungstechnischen Gründen kann es sich als vorteilhaft erweisen, eine Teilmenge des Treibmittels (d) in (a) und die restliche Teilmenge des Treibmittels in (b) oder der Mischung aus (b) und (c) zu emulgieren oder bei Verwendung von verschiedenartigen Treibmitteln (d) kann ein Treibmittel (d) in (a) oder (b) und das andere oder die anderen Treibmittel (d) in der verbleibenden anderen Aufbaukomponente emulgiert werden.

Bei Verwendung von organischen und/oder modifizierten organischen Polyisocyanaten (a) als andere Emulsionsphase finden vorzugsweise aromatische Polyisocyanate, ausgewählt aus der Gruppe 2,4-, 2,6-Toluylen-diisocyanat oder Mischungen der genannten Isomeren, 4,4'-, 2,4'- und 2,2'-Diphenylmethan-diisocyanat oder Mischungen aus mindestens zwei der genannten Isomeren und Mischungen aus Diphenylmethan-diisocyanaten und Polyphenyl-polymethylen-polyisocyanaten, Anwendung. Sofern die organischen Polyisocyanate bei Raumtemperatur kristallin sind, werden sie durch Mischen mit flüssigen Polyisocyanaten und/oder durch geeignete partielle Modifizierung, wie z.B. Carbodiimidisierung und/oder Urethanisierung, verflüssigt.

Als andere Emulsionsphase finden jedoch vorzugsweise die höhermolekularen Verbindungen mit mindestens zwei reaktiven Wasserstoffatomen Verwendung. Insbesondere geeignet sind Polyester-polyole oder deren Gemische mit einer Funktionalität von 2 bis 3 und einem Molekulargewicht von 480 bis 3000 und Polyether-polyole oder deren Gemische mit einer Funktionalität von 2 bis 6 und einem Molekulargewicht von 400 bis 6000, wobei diese zweckmäßigerweise ausgewählt sind aus der Gruppe der Polyoxyethylen-, Polyoxypropylen-, Polyoxypropylen-polyoxyethylen-polyole und Polyoxytetramethylen-glykole oder Mischungen davon.

Die erfindungsgemäßen lagerstabilen, treibmittelhaltigen Emulsionen enthalten somit oder bestehen vorzugsweise aus

i) 1 bis 150 Gew.-Teilen, vorzugsweise 1 bis 70 Gew.-Teilen und insbesondere 5 bis 50 Gew.-Teilen pro 100 Gew.-Teilen von (ii) bzw. (a), (b) oder (b) und (c), mindestens eines niedrigsiedenden, in (ii) bzw. (a), (b) oder (b) und (c) schwer- oder unlöslichen, partiell fluorierten oder perfluorierten, aliphatischen und/oder cycloaliphatischen Kohlenwasserstoffs mit 3 bis 8 Kohlenstoffatomen und/oder Schwefelhexafluorid als Treibmittel (d) bzw. (i),

ii) mindestens einem organischen und/oder modifizierten organischen Polyisocyanat (a) oder mindestens einer höhermolekularen Verbindung mit mindestens zwei reaktiven Wasserstoffatomen (b) oder Mischungen aus (b) und niedermolekularen Kettenverlängerungs- und/oder Vernetzungsmitteln (c) und

iii) 0,01 bis 6,0 Gew.-Teilen, vorzugsweise 0,1 bis 3,5 Gew.-Teilen pro 100 Gew.-Teilen von (ii) bzw. (a), (b) oder (b) und (e) mindestens eines Esters der Formeln

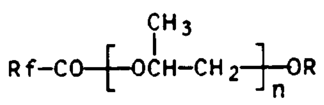

oder

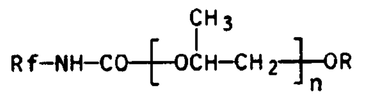 ,

in denen Rf, R und n die vorgenannte Bedeutung haben und

iii) 0 bis 5,0 Gew.-Teilen, vorzugsweise 0,1 bis 3 Gew.-Teilen und insbesondere 0,5 bis 2 Gew.-Teilen pro 100 Gew.-Teilen von (ii) bzw. (a), (b) oder (b) und (c) mindestens eines Polysiloxanes mit Polyetherseitenketten als Schaumstabilisator.

Zur Herstellung der lagerstabilen, treibmittelhaltigen Emulsionen werden die Aufbaukomponenten (a) oder (b) bzw. Mischungen aus (b) und (c) und das Treibmittel (d) in Gegenwart mindestens eines der neuen Fluorkohlenstoff- und Oxypropylenalkylethergruppen gebunden, enthaltenden Carbonsäure- und/oder Carbamidsäureester bei Temperaturen von 0 bis 70°C, vorzugsweise von 20 bis 40°C intensiv vermischt. Als geeignete Mischaggregate hierfür seien beispielhaft genannt: statische Mischer, wie z.B. SMX der Firma Sulzer (Schweiz) oder dynamische Mischer, wie z.B. Ultra-Turrax® der Firma Hanke und Kunkel (BRD). Sofern zur Herstellung der erfindungsgemäßen Emulsionen bei Raumtemperatur gasförmige fluorierte Kohlenwasserstoffe eingesetzt werden, werden diese vor oder während der Emulsionsherstellung durch Anwendung eines Drucks bis 100 bar verflüssigt, so daß insbesondere die Treibmittel Perfluorpropan, Perfluorbutan und Perfluorcyclobutan in der Emulsion unter einem Druck bis 100 bar als flüssige Phase vorliegen.

Zusätzlich zu den vorgenannten Treibmitteln (d) bzw. Treibmittelemulsionen eignet sich auch Wasser als Treibmittel, das mit den organischen, gegebenenfalls modifizierten Polyisocyanaten (a) unter Bildung von Kohlendioxid und Harnstoffgruppen reagiert und dadurch die Druckfestigkeit der Endprodukte beeinflußt. Da die in den Polyester- und Polyether-polyolen als Nebenprodukt enthaltene Wassermenge meist

ausreicht, bedarf es vielfach keiner separaten Wasserzugabe. Sofern jedoch der Polyurethan-Formulierung zusätzlich Wasser einverleibt werden muß, wird dieses üblicherweise in Mengen von 0,05 bis 2 Gew.%, vorzugsweise von 0,1 bis 1, Gew.%, bezogen auf das Gewicht der Aufbaukomponente (b) verwendet.

Die zweckmäßigste Menge an partiell fluorierten und/oder perfluorierten Kohlenwasserstoffen und/oder Schwefelhexafluorid, zur Herstellung der zelligen Polyisocyanat-Polyadditionsprodukte hängt ab von der Dichte, die man erreichen will und der gegebenenfalls eingesetzten Wassermenge. Im allgemeinen liefern Mengen von 1 bis 60 Gew.-Teilen, vorzugsweise 5 bis 40 Gew.-Teilen und insbesondere 10 bis 25 Gew.-Teilen des Treibmittels (d) bezogen auf 100 Gew.-Teile der Aufbaukomponenten (a) bis (c) oder (a) und (b) zufriedenstellende Ergebnisse.

e) Als Katalysatoren (e) zur Herstellung der zelligen Kunststoffe nach dem Polyisocyanat-Polyadditionsverfahren werden insbesondere Verbindungen verwendet, die die Reaktion der Hydroxylgruppen enthaltenden Verbindungen der Komponente (b) und gegebenenfalls (c) mit den organischen, gegebenenfalls modifizierten Polyisocyanaten (a) stark beschleunigen. In Betracht kommen organische Metallverbindungen, vorzugsweise organische Zinnverbindungen, wie Zinn-(II)-salze von organischen Carbonsäuren, z.B. Zinn-(II)-acetat, Zinn-(II)-octoat, Zinn-(II)ethylhexoat und zinn-(II)-laurat und die Dialkylzinn-(IV)-salze von organischen Carbonsäuren, z.B. Dibutyl-zinndiacetat, Dibutylzinn-dilaurat, Dibutylzinn-maleat und Dioctylzinn-diacetat. Die organischen Metallverbindungen werden allein oder vorzugsweise in Kombination mit stark basischen Aminen eingesetzt. Genannt seien beispielsweise Amidine, wie 2,3-Dimethyl-3,4,5,6-tetrahydropyrimidin, tertiäre Amine, wie Triethylamin, Tributylamin, Dimethylbenzylamin, N-Methyl-, N-Ethyl-, N-Cyclohexylmorpholin, N,N,N',N -Tetramethylethylendiamin, N,N,N',N'-Tetramethyl-butandiamin, Pentamethyl-diethylentriamin, Tetramethyl-diaminoethylether, Bis-(dimethylaminopropyl)-harnstoff, Dimethylpiperazin, 1,2-Dimethylimidazol, 1-Aza-bicyclo-(3,3,0)-octan und vorzugsweise 1,4-Diaza-bicyclo-(2,2,2)-octan und Alkanolaminverbindungen, wie Triethanolamin, Triisopropanolamin, N-Methyl- und N-Ethyl-diethanolamin und Dimethylethanolamin.

Als Katalysatoren kommen ferner in Betracht: Tris-(dialkylaminoalkyl)-s-hexahydrotriazine, insbesondere Tris-(N,N-dimethylaminopropyl)-S-hexahydrotriazin, Tetraalkylammoniumhydroxide, wie Tetramethylammoniumhydroxid, Alkalihydroxide, wie Natriumhydroxid und Alkalialkoholate, wie Natriummethylat und Kaliumisopropylat sowie Alkalisalze von langkettigen Fettsäuren mit 10 bis 20 C-Atomen und gegebenenfalls seitenständigen OH-Gruppen. Vorzugsweise verwendet werden 0,001 bis 5 Gew.-%, insbesondere 0,05 bis 2 Gew.-% Katalysator bzw. Katalysatorkombination bezogen auf das Gewicht der Komponente (b).

f) Der Reaktionsmischung zur Herstellung der zelligen Kunststoffe nach dem Polyisocyanat-Polyadditionsverfahren können gegebenenfalls auch noch Hilfsmittel und/oder Zusatzstoffe (f) einverleibt werden. Genannt seien beispielsweise oberflächenaktive Substanzen, Schaumstabilisatoren, Zellregler, Füllstoffe, Farbstoffe, Pigmente, Flammschutzmittel, Hydrolyseschutzmittel, fungistatische und bakteriostatisch wirkende Substanzen.

Als oberflächenaktive Substanzen kommen z.B. Verbindungen in Betracht, welche zur Unterstützung der Homogenisierung der Ausgangsstoffe dienen und gegebenenfalls auch geeignet sind, die Zellstruktur zu regulieren. Genannt seien beispielsweise Emulgatoren, wie die Natriumsalze von Ricinusölsulfaten, oder von Fettsäuren sowie Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure und Ricinolsäure; Schaumstabilisatoren, wie Siloxan-Oxalkylen-Mischpolymerisate und andere Organopolysiloxane, oxethylierte Alkylphenole, oxethylierte Fettalkohole, Paraffinöle, Ricinusöl- bzw. Ricinolsäureester, Türkischrotöl und Erdnußöl und Zellregler, wie Paraffine, Fettalkohole und Dimethylpolysiloxane. Als Schaumstabilisator besonders bewährt haben sich und daher vorzugsweise verwendet werden Polysiloxane mit Polyetherseitenketten und hiervon insbesondere solche mit einem mittleren Molekulargewicht und einer relativ hohen Hydrophilie, da durch diese, wie bereits dargelegt wurde, die Emulgierwirkung der neuen Fluorkohlenstoff- und Oxypropylenalkylethergruppen gebunden enthaltenden Ester verstärkt und die Schaumeigenschaften verbessert werden können. Erwähnenswert ist die feine, gleichförmige Zellstruktur der auf diese Weise hergestellten Polyurethan-Hartschaumstoffe. Derartige hydrophile Polysiloxane mit Polyetherseitenketten sind Handelsprodukte und werden z.B. unter dem Warenzeichen Tegostab® B8406 und B8409 von der Goldschmidt AG, Essen und dem Kennzeichen DC 190 und DG 193 von der Firma Dow, Corning, angeboten. Die oberflächenaktiven Substanzen werden üblicherweise in Mengen von 0,01 bis 5 Gew.-Teilen, bezogen auf 100 Gew.-Teilen der Komponente (b), angewandt.

Als Füllstoffe, insbesondere verstärkend wirkende Füllstoffe, sind die an sich bekannten, üblichen organischen und anorganischen Füllstoffe, Verstärkungsmittel, Beschwerungsmittel, Mittel zur Verbesserung des Abriebverhaltens in Anstrichfarben, Beschichtungsmittel usw. zu verstehen. Im einzelnen seien beispielhaft genannt: anorganische Füllstoffe wie silikatische Mineralien, beispielsweise Schichtsilikate wie Antigorit, Serpentin, Hornblenden, Amphibole, Chrisotil, Zeolithe, Talkum; Metalloxide, wie Kaolin, Aluminiumoxide, Titanoxide und Eisenoxide, Metallsalze wie Kreide, Schwerspat und anorganische Pigmente, wie Cadmiumsulfid, Zinksulfid sowie Glas u.a.. Vorzugsweise verwendet werden Kaolin (China Clay), Aluminiumsilikat und Copräzipitate aus Bariumsulfat und Aluminiumsilikat sowie natürliche und synthetische faserförmige Mineralien wie Wollastonit, Metall- und insbesondere Glasfasern verschiedener Länge, die gegebenenfalls geschlichtet sein können. Als organische Füllstoffe kommen beispielsweise in Betracht: Ruß, Melamin, Kollophonium, Cyclopentadienylharze und Pfropfpolymerisate sowie Cellulosefasern, Polyamid-, Polyacrylnitril-, Polyurethan-, Polyesterfasern auf der Grundlage von aromatischen und/oder aliphatischen Dicarbonsäureestern und insbesondere Kohlenstoffasern.

Die anorganischen und organischen Füllstoffe können einzeln oder als Gemische verwendet werden und werden der Reaktionsmischung vorteilhafterweise in Mengen von 0,5 bis 50 Gew.%, vorzugsweise 1 bis 40 Gew.%, bezogen auf das Gewicht der Komponenten (a) bis (c), einverleibt, wobei jedoch der Gehalt an Matten, Vliesen und Geweben aus natürlichen und synthetischen Fasern Werte bis 80 Gew.% erreichen kann.

Geeignete Flammschutzmittel sind beispielsweise Trikresylphosphat, Tris-2-chlorethylphosphat, Trischlorpropylphosphat und Tris-2,3-dibrompropylphosphat.

Außer den bereits genannten halogensubstituierten Phosphaten können auch anorganische Flammschutzmittel, wie roter Phosphor, Aluminiumoxidhydrat, Antimontrioxid, Arsenoxid, Ammoniumpolyphosphat und Calciumsulfat oder Cyanursäurederivate, wie z.B. Melamin oder Mischungen aus mindestens zwei Flammschutzmitteln, wie z.B. Ammoniumpolyphosphaten und Melamin sowie gegebenenfalls Maisstärke zum Flammfestmachen der Polyisocyanat-polyadditionsprodukte verwendet werden. Im allgemeinen hat es sich als zweckmäßig erwiesen, 5 bis 50 Gew.-Teile, vorzugsweise 5 bis 25 Gew.-Teile der genannten Flammschutzmittel für jeweils 100 Gew.-Teile der Komponente (b) zu verwenden.

Nähere Angaben über die oben genannten anderen üblichen Hilfs- und Zusatzstoffe sind der Fachliteratur, beispielsweise der Monographie von J.H. Saunders und K.C. Frisch "High Polymers" Band XVI, Polyurethanes, Teil 1 und 2, Verlag Interscience Publishers 1962 bzw. 1964, oder dem Kunststoff-Handbuch, Polyurethane, Band VII, Hanserverlag, München, Wien, 1. und 2. Auflage, 1966 und 1983 zu entnehmen.

Zur Herstellung der zelligen Urethangruppen enthaltenden Kunststoffe werden die organischen Polyisocyanate (a), höhermolekularen Verbindungen mit mindestens zwei reaktiven Wasserstoffatomen (b) und gegebenenfalls Kettenverlängerungs- und/oder Vernetzungsmittel (c) in solchen Mengen zur Umsetzung gebracht, daß das Äquivalenz-Verhältnis von NCO-Gruppen der Polyisocanate (a) zur Summe der reaktiven Wasserstoffatome der Komponenten (b) und gegebenenfalls (c) 0,85 bis 1,25:1, vorzugsweise 0,95 bis 1,15:1 beträgt. Falls die zelligen Kunststoffe zumindest teilweise Isocyanuratgruppen gebunden enthalten, wird üblicherweise ein Verhältnis von NCO-Gruppen der Polyisocyanate (a) zur Summe der reaktiven Wasserstoffatome der Komponente (b) und gegebenenfalls (c) von 1,5 bis 60:1, vorzugsweise 1,5 bis 8:1 angewandt.

Die zellhaltigen Kunststoffe aus Polyisocyanat-polyadditionsprodukten, wie zelligen Elastomeren oder vorzugsweise Schaumstoffe, insbesondere Hartschaumstoffe, werden vorteilhafterweise nach dem one shot-Verfahren, beispielsweise mit Hilfe der Reaktionsspritzguß-, Hochdruck- oder Niederdruck-Technik in offenen oder geschlossenen Formwerkzeugen, beispielsweise temperierbaren, metallischen Formwerkzeugen hergestellt. Als besonders vorteilhaft hat es sich erwiesen, nach dem Zweikomponenten-Verfahren zu arbeiten und die Aufbaukomponenten (b), (d), (e) und gegebenenfalls (c) und (f) in der Komponente (A) zu vereinigen und als Komponente (B) die organischen Polyisocyanate, modifizierten Polyisocyanate (a) oder Mischungen aus den genannten Polyisocyanaten und gegebenenfalls Treibmittel (d) zu verwenden.

Die Ausgangskomponenten werden bei einer Temperatur von 15 bis 90°C, vorzugsweise von 20 bis 35°C gemischt und in das offene oder gegebenenfalls unter erhöhtem Druck in das geschlossene Formwerkzeug eingebracht. Die Vermischung kann, wie bereits dargelegt wurde, mechanisch mittels eines Rührers oder einer Rührschnecke oder unter hohem Druck im sogenannten Gegenstrominjektionsverfahren durchgeführt werden. Die Formwerkzeugtemperatur beträgt zweckmäßigerweise 20 bis 90°C, vorzugsweise 30 bis 60°C und insbesondere 45 bis 50°C.

Die nach dem erfindungsgemäßen Verfahren hergestellten zelligen Elastomeren besitzen Dichten von ungefähr 0,76 bis 1,0 g/cm³, vorzugsweise von 0,9 bis 1,0 g/cm³, wobei die Dichte von füllstoffhaltigen Produkten höhere Werte, z.B. bis 1,4 g/cm³ und mehr erreichen kann. Formkörper aus derartigen zelligen Elastomeren finden Verwendung in der Automobilindustrie, beispielsweise als Kopfstützen, Außenteile,

wie z.B. Heckspoiler und Stoßfänger und Innenauskleidungen sowie als Schuhsohlen.

Die nach dem erfindungsgemäßen Verfahren hergestellten weichelastischen, halbharten und harten Schaumstoffe sowie die entsprechenden Integralschaumstoffe weisen eine Dichte von 0,02 bis 0,75 g/cm³ auf, wobei die Dichte der Schaumstoffe vorzugsweise 0,025 bis 0,24 g/cm³ und insbesondere 0,03 bis 0,1 g/cm³ und die Dichte der Integralschaumstoffe vorzugsweise 0,08 bis 0,75 g/cm³, und insbesondere 0,24 bis 0,6 g/cm³ betragen. Die Schaumstoffe und Integralschaumstoffe werden z.B. verwendet in der Fahrzeug-, z.B. Automobil-, Flugzeug- und Schiffbauindustrie, der Möbel und Sportartikelindustrie als beispielsweise Polstermaterialien, Gehäuseteile, Skiinnenschuh, Skikern u.a. Besonders eignen sie sich als Isolationsmaterial im Bau- und Kühlschranksektor.

Die erfindungsgemäßen lagerstabilen, treibmittelhaltigen Emulsionen finden Verwendung zur Herstellung zelligen Kunststoffen, vorzugweise von Urethan- oder Urethan- und Isocyanuratgruppen enthaltenden Schaumstoffen, insbesondere Hartschaumstoffen, und Urethangruppen enthaltenden zelligen Elastomeren nach dem Polyisocyanat-Polyadditionsverfahren.

## Beispiele

a) Herstellung der Fluorkohlenstoff- und Oxypropylenalkylethergruppen gebunden enthaltenden Carbonsäure- oder Carbamidsäureester

### Beispiel 1

| | |
|---|---|
| 33,6 Gew.-Teile | Polyoxypropylenglykolmonomethylether mit einer OH-Zahl von 144, hergestellt durch anionische Polyaddition von 1.2-Propylenoxid an Methanol, wurden auf 0°C abgekühlt und unter intensivem Rühren mit |
| 20 Gew.-Teilen | Perfluorbuttersäurechlorid innerhalb von 5 Minuten versetzt. Nach einer Rührzeit von 10 Minuten ließ man zu der Reaktionsmischung innerhalb von 20 Minuten |
| 13,6 Gew.-Teile | Pyridin zu tropfen. Es entstand ein farbloser, kristalliner Niederschlag. Das Reaktionsgemisch wurde anschließend unter weiterem Rühren auf 80°C erwärmt und bei dieser Temperatur die Reaktion in 10 Minuten zu Ende geführt. |
| Nach Zugabe von | |
| 250 Gew.-Teilen | Eiswasser und verdünnter Salzsäure wurde das Reaktionsgemisch mit |
| 100 Gew.-Teilen | Ethylacetat ausgeschüttelt, die Ethylacetatfraktion zweimal mit Natriumhydrogencarbonatlösung gewaschen, getrocknet und danach das Ethylacetat bei 40°C unter vermindertem Druck (ungef. 100 mbar) abdestilliert. Als Rückstand erhielt man |
| 54,4 Gew.-Teile | (93 Gew.-% der Th.) des gewünschten Polyoxypropylen glykolmonomethylether-perfluorbuttersäureesters in Form einer klaren Flüssigkeit mit einem Fluorgehalt von 22,3 Gew.-%. Der theoretische Fluorgehalt beträgt be rechnet: 22,4 Gew.-% |

### Beispiel 2

Man verfuhr analog den Angaben des Beispiels 1, verwendete jedoch folgende Einsatzstoffe und -mengen:

| | |
|---|---|
| 32,3 Gew.-Teile | Polyoxypropylenglykolmonomethylether mit einer Hydroxyl zahl von 79, hergestellt durch anionische Polyaddition von 1.2-Propylenoxid an Methanol, |
| 18,0 Gew.-Teile | Perfluorhexylacetylchlorid ($C_6F_{13}$-$CH_2$-COCl) und |
| 7,2 Gew.-Teile | Pydridin |
| Man erhielt | |
| 44,2 Gew.-Teile | (91 Gew.-% der Th.) des gewünschten Polyoxypropylenglykolmonomethylether-perfluorhexylessigsäureesters in Form einer rotbraunen, leicht viskosen Flüssigkeit mit einem Fluorgehalt von 22,2 Gew.-%. Der theoretische Fluorgehalt beträgt berechnet 23,0 Gew.-%. |

### Beispiel 3

Man verfuhr analog den Angaben des Beispiels 1, verwendete jedoch die folgenden Ausgangsstoffe und -mengen:

| | |
|---|---|
| 47,4 Gew.-Teile | Polyoxypropylenglykolmonomethylether mit einer Hydroxylzahl von 79, hergestellt durch anionische Polyaddition von 1.2-Propylenoxid an Methanol, |
| 15,4 Gew.-Teile | Pentafluorbenzoesäurechlorid und |
| 10,6 Gew.-Teile | Pyridin. |
| Man erhielt | |
| 56,5 Gew.-Teile | (94 Gew.-% der Th.) des gewünschten Polyoxypropylenglykolmonomethylether-pentafluorbenzoesäureesters in Form einer rotbraunen Flüssigkeit mit einem Fluorgehalt von 10,1 Gew.-%. Der theoretische Fluorgehalt beträgt berechnet 10,5 Gew.-%. |

Beispiel 4

Zu 138 Gew.-Teilen eines Polyoxypropylenglykolmonomethylethers mit einer Hydroxylzahl von 41, hergestellt durch anionische Polyaddition von 1.2-Propylenoxid an Methanol, fügte man unter Rühren unter einer Atmosphäre aus trockenem Stickstoff bei 25°C innerhalb eines Zeitraums von 20 Minuten 18,7 Gew.-Teile 3-Trifluormethylphenylisocyanat.

Das Reaktionsgemisch wurde danach auf 70°C erwärmt und die Reaktion bei dieser Temperatur durch zweistündiges Rühren zu Ende geführt. Man erhielt den N-(3-Trifluormethylphenyl)-carbamidsäurepolyoxypropylenglykolmonomethyletherester in Form einer klaren, gelblichen Flüssigkeit mit einem Fluorgehalt von 3,6 Gew.-%. Der theoretische Fluorgehalt beträgt berechnet 3,64 Gew.-%.

b) Herstellung lagerstabiler, treibmittelhaltiger Emulsionen

Beispiel 5

Eine Mischung, die bestand aus

| | |
|---|---|
| 60 Gew.-Teilen | eines Polyoxypropylen-polyols mit einer Hydroxylzahl von 400, hergestellt durch anionische Polyaddition von 1.2-Propylenoxid an Saccharose als Startermolekül, |
| 40 Gew.-Teilen | Perfluorhexan und |
| 0,5 Gew.-Teilen | eines Polyoxypropylenglykolmonomethylether-perfluorbuttersäureesters, hergestellt analog den Angaben des Beispiels 1 aus einem Polyoxypropylenglykolmonomethylether mit einer Hydroxylzahl von 79 und Perfluorbuttersäurechlorid, |

wurde bei 23°C mit einem schnellaufenden Rührer (2000 Upm) in 30 Sekunden intensiv gemischt.

Man erhielt eine Emulsion, die kein freies, nicht emulgiertes Perfluorhexan enthielt und nach einer Lagerzeit von einer Woche bei Raumtemperatur noch stabil war.

Beispiel 6

Man verfuhr analog den Angaben des Beispiels 5, fügte der Mischung jedoch vor der Emulgierung zusätzlich 1 Gew.-Teil eines Schaumstabilisators auf der Grundlage eines hydrophilen Polysiloxans mit Polyetherseitenketten (Tegostab® B8409, der Goldschmidt AG, Essen) bei.

Man erhielt eine Emulsion, die nach mehr als einer Woche lagern bei Raumtemperatur stabil war und keinerlei freies, entmischtes, nicht emulgiertes Perfluorhexan enthielt.

Beispiel 7

Verfuhr man analog den Angaben des Beispiels 5, verwendete jedoch anstelle des Polyoxypropylenglykolmonomethylether-perfluorbuttersäureesters 0,5 Gew.-Teile Polyoxypropylenglykolmonomethylether-pentafluorbenzoesäureester, hergestellt nach den Angaben des Beispiels 3, so erhielt man eine stabile Emulsion, die nach einer Lagerzeit von einer Woche bei Raumtemperatur keinerlei Anzeichen einer Entmischung zeigte und kein freies Perfluorhexan enthielt.

Beispiel 8

Eine Mischung, die bestand aus

| | |
|---|---|
| 60 Gew.-Teilen | eines Polyoxypropylen-polyols mit einer Hydroxylzahl von 400, hergestellt durch anionische Polyaddition von 1.2-Propylenoxid an Saccharose als Startermolekül, |
| 40 Gew.-Teilen | Perfluorhexan, |
| 1,0 Gew.-Teilen | eines hydrophilen Polysiloxan mit Polyetherseitenketten (Tegostab® B8406 der |

| | |
|---|---|
| | Goldschmidt AG, Essen) und |
| 0,5 Gew.-Teilen | Polyoxypropylenglykolmonomethylether-pentafluorbenzoesäureesters, hergestellt nach den Angaben des Beispiels 3 |

wurde bei 23°C mit einem schnellaufenden Rührer (2000 Upm) in 30 Sekunden intensiv gemischt.

Man erhielt eine stabile Emulsion, die kein freies, nicht emulgiertes Perfluorhexan enthielt, und nach mehr als einer Woche lagern bei Raumtemperatur keinerlei Anzeichen einer Phasentrennung zeigte und kein entmischtes, freies Perfluorhexan aufwies.

Vergleichsversuch I

Eine Mischung, die bestand aus

| | |
|---|---|
| 60 Gew.-Teilen | eines Polyoxypropylen-polyols mit einer Hydroxylzahl von 400, hergestellt durch anionische Polymerisation von 1.2-Propylenoxid an Saccharose als Startermolekül, |
| 40 Gew.-Teilen | Perfluorhexan und |
| 1,0 Gew.-Teilen | eines hydrophilen Polysiloxans mit Polyetherseitenketten (Tegostab® B8409 der Goldschmidt AG, Essen) |

wurde bei 23°C mit einem schnellaufenden Rührer (2000 Upm) 30 Sekunden lang intensiv gemischt.

Es entstand eine Emulsion, die ungefähr 30 Gew.-Teile (ca. 75 Gew.-%) des eingesetzten Perfluorhexans emulgiert enthielt. Die gebildete Emulsion wurde vom nicht emulgierten Perfluorhexan abgetrennt und bei 23°C gelagert. Innerhalb von 24 Stunden begann sich die Emulsion zu entmischen und Perfluorhexan abzuscheiden.

c) Herstellung eines Polyurethan-Hartschaumstoffs

Beispiele 9 bis 16

Allgemeine Herstellungsvorschrift

Einer Mischung, die bestand aus

| | |
|---|---|
| 76,2 Gew.-Teilen | eines Polyoxypropylen-polyols mit einer Hydroxylzahl von 400, hergestellt durch anionische Polyaddition von 1.2-Propylenoxid an Saccharose als Startermolekül, |
| 1,6 Gew.-Teilen | Wasser, |
| 2,1 Gew.-Teilen | N,N-Dimethyl-cyclohexylamin und |
| 2.0 Gew.-Teilen | eines hydrophilen Polysiloxans mit Polyetherseitenketten (Tegostab® B8409 der Goldschmidt AG, Essen) |

wurden zunächst die nachfolgend genannten Fluorkohlenstoff- und Oxypropylenmethylethergruppen gebunden enthaltende Carbonsäure- oder Carbamidsäureester in den genannten Mengen einverleibt und danach wurden in der erhaltenen Mischung

| | |
|---|---|
| 20 Gew.-Teile | Perfluorhexan |

mit Hilfe eines schnellaufenden Rührers (2000 UpM) bei 25°C in 30 Sekunden emulgiert.

Die erhaltenen Emulsionen wurden bei 23°C mit 109 Gew.-Teilen einer Mischung aus Diphenylmethandiisocyanaten und Polyphenyl-polymethylen-polyisocyanaten (Roh-MDI) mit einem NCO-Gehalt von 31 Gew.-% intensiv gemischt, die erhaltenen Reaktionsmischungen in ein offenes, eimerförmiges Gefäß eingefüllt und dort frei aufschäumen gelassen.

Man erhielt sehr feinzellige Polyurethan-Hartschaumstoffe der Dichte 35 g/l mit gleichförmiger Zellstruktur, wie sie beim konventionellen Schaumprozeß mit Trichlorfluormethan als Treibmittel nicht erreicht werden konnten.

Die erhaltenen Perfluorhexan als Treibmittel enthaltenden Emulsionen wurden bei 23°C gelagert und zeigten nach 8 Tagen keinerlei Anzeichen einer Entmischung oder Perfluorhexan Absonderung.

Nach dieser Zeit wurden sie erneut mit 109 Gew.-Teilen Roh-MDI gemischt und nach der beschriebenen Methode aufschäumen gelassen.

Die erhaltenen Polyurethan-Hartschaumstoffe zeigten keine Veränderung der Zellstruktur.

Tabelle

| Beispiele | Emulgator Art | Menge [Gew.-Teile] | Schaum-struktur |
|---|---|---|---|
| 9 | Carbonsäureester nach Beispiel 1 | 1,64 | hervorragend feinzellig |
| 10 | Carbonsäureester, hergestellt analog Beispiel 1 aus Perfluorbuttersäurechlorid und einem Polyoxypropylenglykolmonomethylether der Hydroxylzahl 79 | 1,64 | sehr feinzellig |
| 11 | Carbonsäureester, hergestellt analog Beispiel 2 aus Perfluorhexylacetylchlorid und einem Polyoxypropylenglykolmonomethylether der Hydroxylzahl 144 | 0,26 | hervorragend feinzellig |
| 12 | Carbonsäureester nach Beispiel 2 | 0,26 | sehr feinzellig |
| 13 | Carbonsäureester, hergestellt analog Beispiel 2 aus Perfluorhexylacetylchlorid und einem Polyoxypropylenglykolmonomethylether der Hydroxylzahl 41 | 0,26 | sehr feinzellig |
| 14 | Carbonsäureester nach Beispiel 3 | 0,26 | sehr feinzellig |
| 15 | Carbamidsäureester, hergestellt analog Beispiel 4 aus 3-Trifluormethyl-phenylisocyanat und einem Polyoxypropylenglykolmonomethylether der Hydroxylzahl 144 | 0,26 | sehr feinzellig |
| 16 | Carbamidsäureester, hergestellt analog Beispiel 4 aus 3-Trifluormethyl-phenylisocyanat und einem Polyoxypropylenglykolmonomethylether der Hydroxylzahl 79 | 0,55 | sehr feinzellig |

Vergleichsversuch II

In eine Mischung, die bestand aus

| | |
|---|---|
| 76,2 Gew.-Teilen | eines Polyoxypropylen-polyols mit einer Hydroxylzahl von 400, hergestellt durch anionische Polyaddition von 1.2-Propylenoxid an Saccharose als Startermolekül, |
| 1,6 Gew.-Teilen | Wasser, |
| 2,1 Gew.-Teilen | N,N-Dimethyl-cyclohexylamin und |
| 2.0 Gew.-Teilen | eines hydrophilen Polysiloxans mit Polyetherseitenketten (Tegostab® B8409 der Goldschmidt AG, Essen) |

wurden mittels eines hochtourigen Rührens (2000 Upm) bei 25°C in 30 Sekunden

20 Gew.-Teile Perfluorhexan

emulgiert.

Die entstandene Emulsion wurde sofort bei 23°C mit Roh-MDI mit einem NCO-Gehalt von 31 Gew.-% intensiv vermischt, die erhaltene Reaktionsmischung in ein offenes eimerförmiges Gefäß eingefüllt und dort frei aufschäumen gelassen.

Man erhielt einen relativ grobzelligen Polyurethan-Hartschaumstoffe der Dichte 50 g/l.

Wurde der Schaumstabilisator Tegostab® B8409 durch andere Schaumstabilisatoren auf der Grundlage von Siloxanen, wie z.B. Tegostab® B1903, B8406, B8422 oder DC190 oder DC193 der Firma Dow Corning, ersetzt, so wurden Polyurethan-Hartschaumstoffe mit einer noch schlechteren Zellstruktur erhalten.

Beispiel 17

Man verfuhr analog den Angaben des Beispiels 10, verwendete jedoch anstelle von Perfluorhexan 30 Gew.-Teile Heptafluorpropan als Treibmittel.

Man erhielt einen feinzelligen Polyurethan-Hartschaumstoff der Dichte 23 g/Liter.

Beispiel 18

Man verfuhr analog den Angaben des Beispiels 11, setzte jedoch anstelle von Perfluorhexan

17 Gew.-Teile Perfluorpentan als Treibmittel ein.

Man erhielt einen sehr feinzelligen Polyurethan-Hartschaumstoff mit der Dichte 35 g/Liter.

Beispiel 19

In eine Mischung, die bestand aus

| | |
|---|---|
| 58,4 Gew.-Teilen | eines Polyoxypropylen-polyols mit der Hydroxylzahl 490, hergestellt mit Sorbitol als Startermolekül, |
| 8,0 Gew.-Teilen | Glycerin, |
| 11,1 Gew.-Teilen | Dipropylenglykol, |
| 1,9 Gew.-Teilen | Diethanolamin, |
| 14,0 Gew.-Teilen | β-Trichlorethylphosphat, |
| 1,6 Gew.-Teilen | N,N-Dimethylcyclohexylamin, |
| 3,2 Gew.-Teilen | Polysiloxan mit Polyetherseitenketten (Tegostab® B 8406 der Goldschmidt AG, Essen) und 1,8 Gew.-Teilen Polyoxypropylenglykolmonomethylether-perfluorbutter säureester, hergstellt nach Angaben des Beispiels 1 |

wurden bei 23°C unter Rühren in einem druckfest verschlossenen Gefäß 21 Gew.-Teile Heptafluorpropan emulgiert. Die stabile Emulsion wurde anschließend bei 23°C mit

155 Gew.-Teilen einer Mischung aus Diphenylmethan-diisocyanaten und Polyphenyl-polymethylen-polyisocyanaten mit einem NCO-Gehalt von 31 Gew.-% intensiv gemischt, die Reaktionsmischung in ein offenes Formwerkzeug eingefüllt und dort frei aufschäumen gelassen.

Man erhielt einen feinzelligen Polyurethan-Hartschaumstoff mit der Dichte 50 g/Liter.

Beispiel 20

In eine Mischung, die bestand aus

| | |
|---|---|
| 70,4 Gew.-Teilen | eines mit 1,3-Propandiol gestarteten Polyoxypropylen(80)-polyoxyethylen(20)-glykols der Hydroxylzahl von 30, |
| 17,5 Gew.-Teilen | eines mit Glycerin gestarteten Polyoxypropylen(80)-polyoxyethylen(20)-polyols mit |

| | |
|---|---|
| | der Hydroxylzahl von 35, |
| 9,2 Gew.-Teilen | 1,4-Butandiol, |
| 0,1 Gew.-Teilen | eines Polysiloxans mit Polyetherseitenketten (DG 193 der Firma Dow Corning), |
| 1,8 Gew.-Teilen | einer 25 %igen Lösung von Triethylendiamin in 1,4-Butandiol, |
| 0,02 Gew.-Teilen | Dibutylzinndilaurat und |
| 1,0 Gew.-Teilen | Polyoxypropylenglykolmonomethylether-perfluorhexylacetat hergestellt nach Angaben des Beispiels 2 |

wurden bei 23°C unter Rühren in einem druckfest verschlossenen Gefäß 2 Gew.-Teile Schwefelhexafluorid emulgiert. Die erhaltene Emulsion wurde danach bei 23°C mit
53 Gew.-Teilen eines Urethangruppen enthaltenden Polyisocyanats mit einem NCO-Gehalt von 23 Gew.-%, hergestellt durch Umsetzung von 4,4'-Diphenylmethan-diisocyanat mit einem Polyoxypropylen-glykol vom Molekulargewicht 400,
intensiv gemischt, die Reaktionsmischung in ein offenes Formwerkzeug eingefüllt und dort frei aufschäumen gelassen.

Man erhielt einen mikrozelligen Polyurethanschaumstoff mit der Dichte 300 g/Liter.

**Patentansprüche**

1. Neue Fluorkohlenstoff- und Oxypropylenalkylethergruppen gebunden enthaltende Ester der Formeln

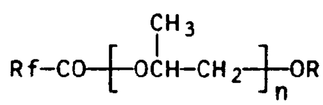

oder

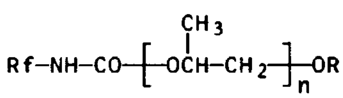

in denen bedeuten:

| | |
|---|---|
| Rf | eine lineare oder verzweigte, partiell fluorierte oder perfluorierte Alkylgruppe mit 2 bis 10 Kohlenstoffatomen, eine partiell fluorierte oder perfluorierte Cycloalkylgruppe mit 4 bis 8 Kohlenstoffatomen, eine Perfluorphenylgruppe oder eine Perfluoralkylphenylgruppe mit 1 bis 6 Kohlenstoffatomen im Perfluoralkylrest, |
| R | eine lineare oder verzweigte Alkylgruppe mit 1 bis 4 Kohlenstoffatomen und |
| n | eine ganze Zahl von 2 bis 70. |

2. Neue Fluorkohlenstoff- und Oxypropylenalkylethergruppen gebunden enthaltende Ester nach Anspruch 1, dadurch gekennzeichnet, daß die Gruppe Rf eine Fluorgehalt von mindestens 40 Gew.-% besitzt.

3. Neue Fluorkohlenstoff- und Oxypropylenalkylethergruppen gebunden enthaltende Ester nach Anspruch 1, dadurch gekennzeichnet, daß die Gruppe Rf besteht aus einer der Gruppen $C_3F_7$-, $C_6F_{13}$-$CH_2$-, $C_6F_5$- oder $CF_3$-$G_6H_4$-.

4. Neue Fluorkohlenstoff- und Oxypropylenalkylethergruppen gebunden enthaltende Ester nach einem der Ansprüche 1 bis 3, dadurch gekennzeichnet, daß die Polyoxypropylengruppe aus 5 bis 25 Oxypropyleneinheiten besteht.

5. Neue Fluorkohlenstoff- und Oxypropylenalkylethergruppen gebunden enthaltende Ester nach einem der Ansprüche 1 bis 4, dadurch gekennzeichnet, daß die Gruppe R aus einer Methylgruppe besteht.

6. Lagerstabile, treibmittelhaltige Emulsionen, die enthalten
i) mindestens einen in (ii) schwer- oder unlöslichen, partiell fluorierten oder perfluorierten, aliphatischen und/oder cycloaliphatischen Kohlenwasserstoff mit 3 bis 8 Kohlenstoffatomen und/oder Schwefelhexafluorid,
ii) mindestens ein organisches und/oder modifiziertes organisches Polyisocyanat oder mindestens eine höhermolekulare Verbindung mit mindestens zwei reaktiven Wasserstoffatomen oder eine Mischung aus mindestens einer höhermolekularen Verbindung mit mindestens zwei reaktiven Wasserstoffatomen und mindestens einem niedermolekularen Kettenverlängerungs- und/oder Vernetzungsmittel und
iii) mindestens einen Ester der Formeln

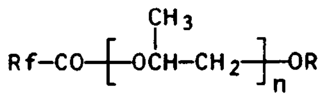

oder

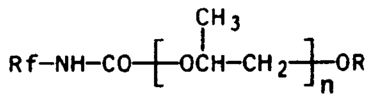

,

in denen Rf, R und n die im Anspruch 1 genannte Bedeutung haben.

**7.** Lagerstabile, treibmittelhaltige Emulsionen, die bestehen aus
i) 1 bis 150 Gew.-Teilen pro 100 Gew.-Teilen von (ii) mindestens eines in (ii) schwer- oder unlöslichen, partiell fluorierten oder perfluorierten, aliphatischen und/oder cycloaliphatischen Kohlenwasserstoffs mit 3 bis 8 Kohlenstoffatomen und/oder Schwefelhexafluorid,
ii) mindestens einer höhermolekularen Verbindung mit mindestens zwei reaktiven Wasserstoffatomen oder einer Mischung aus mindestens einer höhermolekularen Verbindung mit mindestens zwei reaktiven Wasserstoffatomen und mindestens einem niedermolekularen Kettenverlängerungs- und/oder Vernetzungsmittel,
iii) 0,01 bis 6,0 Gew.-Teilen pro 100 Gew.-Teilen von (ii) mindestens eines Esters der Formeln

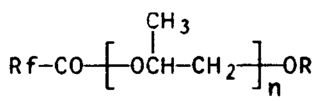

oder

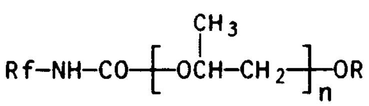

,

in denen Rf, R und n die im Anspruch 1 genannte Bedeutung haben und
iiii) 0 bis 5,0 Gew.-Teilen pro 100 Gew.-Teilen von (ii) mindestens eines Polysiloxanes mit Polyetherseitenketten als Schaumstabilisator.

**8.** Lagerstabile, treibmittelhaltige Emulsionen nach Anspruch 6 oder 7, dadurch gekennzeichnet, daß die fluorierten Kohlenwasserstoffe (i) ausgewählt sind aus der Gruppe Perfluorpentan, Perfluorcyclopentan, Perfluorhexan, Perfluorcyclohexan, Perfluorheptan und Perfluoroctan.

**9.** Lagerstabile, treibmittelhaltige Emulsionen nach Anspruch 6 oder 7, dadurch gekennzeichnet, daß die fluorierten Kohlenwasserstoffe (i) ausgewählt sind aus der Gruppe der unter einem Druck bis 100 bar verflüssigten Gase Perfluorpropan, Perfluorbutan und Perfluorcyclobutan.

**10.** Lagerstabile, treibmittelhaltige Emulsionen nach Anspruch 6 oder 7, dadurch gekennzeichnet, daß die

fluorierten Kohlenwasserstoffe (i) bestehen aus Hexafluorpropan und/oder Heptafluorpropan.

11. Lagerstabile, treibmittelhaltige Emulsionen nach einem der Ansprüche 6 bis 10, dadurch gekennzeichnet, daß die höhermolekularen Verbindungen mit mindestens zwei reaktiven Wasserstoffatomen bestehen aus mindestens einem Polyester-polyol mit einer Funktionalität von 2 bis 3 und einem Molekulargewicht von 480 bis 3000 oder mindestens einem Polyether-polyol mit einer Funktionalität von 2 bis 6 und einem Molekulargewicht von 380 bis 8000.

12. Lagerstabile, treibmittelhaltige Emulsionen nach einem der Ansprüche 6 bis 11, dadurch gekennzeichnet, daß der Ester (iii) ausgewählt ist aus Verbindungen der Formeln

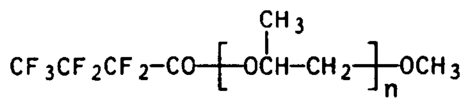

,

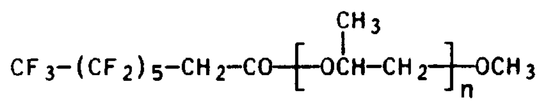

,

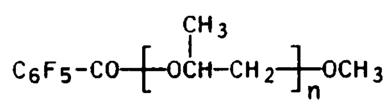

und

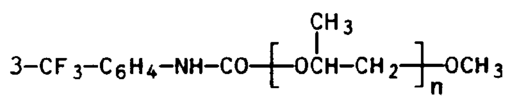

,

in denen n eine Zahl von 6 bis 24 bedeutet.

13. Verwendung von lagerstabilen, treibmittelhaltigen Emulsionen, die enthalten
i) mindestens einen in (ii) schwer- oder unlöslichen, partiell fluorierten oder perfluorierten, aliphatischen und/oder cycloaliphatischen Kohlenwasserstoff mit 3 bis 8 Kohlenstoffatomen und/oder Schwefelhexafluorid,
ii) mindestens ein organisches und/oder modifiziertes organisches Polyisocyanat oder mindestens eine höhermolekulare Verbindung mit mindestens zwei reaktiven Wasserstoffatomen oder eine Mischung aus mindestens einer höhermolekularen Verbindung mit mindestens zwei reaktiven Wasserstoffatomen und mindestens einem niedermolekularen Kettenverlängerungs- und/oder Vernetzungsmittel und
iii) mindestens einen Ester der Formeln

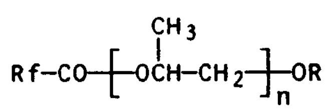

oder

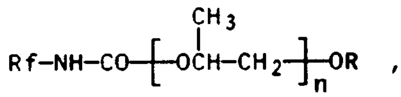

in denen Rf, R und n die im Anspruch 1 genannte Bedeutung haben und

iiii) gegebenenfalls mindestens ein Polysiloxan mit Polyetherseitenketten zur Herstellung von zellhaltigen Kunststoffen, vorzugsweise Polyurethan-Hartschaumstoffen, nach dem Polyisocyanat-Polyadditionsverfahren.

## Claims

1. A novel ester which contains bonded fluorocarbon and oxypropylene alkyl ether groups and is of the formula

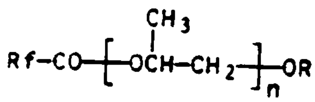

or

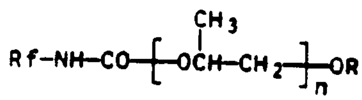

where
Rf is straight-chain or branched, partially fluorinated or perfluorinated alkyl of 2 to 10 carbon atoms, partially fluorinated or perfluorinated cycloalkyl of 4 to 8 carbon atoms,
perfluorophenyl or
perfluoroalkylphenyl where the perfluoroalkyl radical is of 1 to 6 carbon atoms,
R is straight-chain or branched alkyl of 1 to 4 carbon atoms and
n is an integer from 2 to 70.

2. A novel ester containing bonded fluorocarbon and oxypropylene alkyl ether groups, as claimed in claim 1, wherein the group Rf has a fluorine content of not less than 40% by weight.

3. A novel ester containing bonded fluorocarbon and oxypropylene alkyl ether groups, as claimed in claim 1, wherein the group Rf consists of one of the groups $C_3F_7$-, $C_6F_{13}$-$CH_2$-, $C_6F_5$- or $CF_3$-$C_6H_4$-.

4. A novel ester containing bonded fluorocarbon and oxypropylene alkyl ether groups, as claimed in claim 1 or 2 or 3, wherein the polyoxypropylene group consists of from 5 to 25 oxypropylene units.

5. A novel ester containing bonded fluorocarbon and oxypropylene alkyl ether groups, as claimed in claim 1 or 2 or 3 or 4, wherein R is methyl.

6. A blowing agent-containing emulsion which has a long shelf life and contains
   i) at least one partially fluorinated or perfluorinated aliphatic and/or cycloaliphatic hydrocarbon of 3 to 8 carbon atoms which is sparingly soluble or insoluble in (ii), and/or sulfur hexafluoride,
   ii) at least one organic and/or modified organic polyisocyanate and at least one relatively high molecular weight compound having at least two reactive hydrogen atoms or a mixture of at least one relatively high molecular weight compound having at least two reactive hydrogen atoms and at least one low molecular weight chain extender and/or crosslinking agent and
   iii) at least one ester of the formula

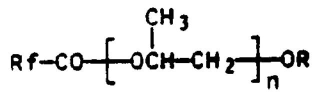

or

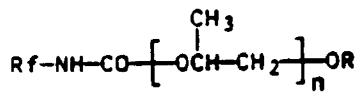,

where Rf, R and n have the meanings stated in claim 1.

7. A blowing agent-containing emulsion which has a long shelf life and consists of
i) from 1 to 150 parts by weight, per 100 parts by weight of (ii) of at least one partially fluorinated or perfluorinated aliphatic and/or cycloaliphatic hydrocarbon of 3 to 8 carbon atoms which is sparingly soluble or insoluble in (ii), and/or sulfur hexafluoride,
ii) at least one relatively high molecular weight compound having at least two reactive hydrogen atoms or a mixture of at least one relatively high molecular weight compound having at least two reactive hydrogen atoms and at least one low molecular weight chain extender and/or crosslinking agent,
iii) from 0.01 to 6.0 parts by weight, per 100 parts by weight of (ii), of at least one ester of the formula

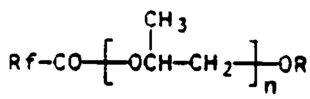

or

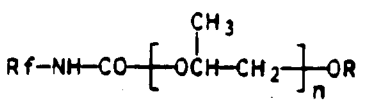,

where Rf, R and n have the meanings stated in claim 1, and
iiii) from 0 to 5.0 parts by weight, per 100 parts by weight of (ii), of at least one polysiloxane having polyether side chains as a foam stabilizer.

8. A blowing agent-containing emulsion having a long shelf life, as claimed in claim 6 or 7, wherein the fluorinated hydrocarbons (i) are selected from the group consisting of perfluoropentane, perfluorocyclopentane, perfluorohexane, perfluorocyclohexane, perfluoroheptane and perfluorooctane.

9. A blowing agent-containing emulsion having a long shelf life, as claimed in claim 6 or 7, wherein the fluorinated hydrocarbons (i) are selected from the group consisting of the gases perfluoropropane, perfluorobutane and perfluorocyclobutane, which are liquefied under a pressure of up to 100 bar.

10. A blowing agent-containing emulsion having a long shelf life, as claimed in claim 6 or 7, wherein the fluorinated hydrocarbons (i) consist of hexafluoropropane and/or heptafluoropropane.

11. A blowing agent-containing emulsion having a long shelf life, as claimed in claim 6 or 7 or 8 or 9 or 10, wherein the relatively high molecular weight compounds having at least two reactive hydrogen atoms consist of at least one polyesterpolyol having a functionality of 2 or 3 and a molecular weight of from 480 to 3,000, or at least one polyetherpolyol having a functionality of 2 to 6 and a molecular weight of from 380 to 8,000.

12. A blowing agent-containing emulsion having a long shelf life, as claimed in claim 6 or 7 or 8 or 9 or 10 or 11, wherein the ester (iii) is selected from compounds of the formulae

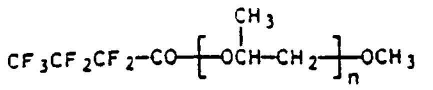

,

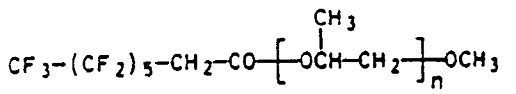

,

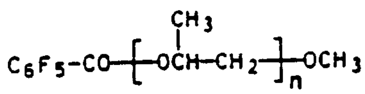

and

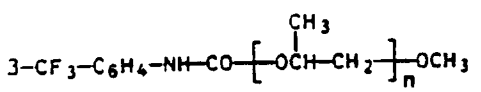

,

where n is from 6 to 24.

13. Use of a blowing agent-containing emulsion which has a long shelf life and contains
    i) at least one partially fluorinated or perfluorinated aliphatic and/or cycloaliphatic hydrocarbon of 3 to 8 carbon atoms which is sparingly soluble or insoluble in (ii), and/or sulfur hexafluoride,
    ii) at least one organic and/or modified organic polyisocyanate and at least one relatively high molecular weight compound having at least two reactive hydrogen atoms or a mixture of at least one relatively high molecular weight compound having at least two reactive hydrogen atoms and at least one low molecular weight chain extender and/or crosslinking agent and
    iii) at least one ester of the formula

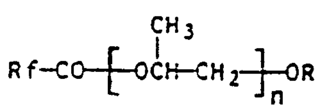

or

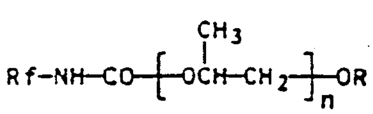

,

where Rf, R and n have the meanings stated in claim 1, and
iiii) if required, at least one polysiloxane having polyether side chains
for the preparation of cellular plastics, preferably rigid polyurethane foams, by the polyisocyanate polyaddition method.

## Revendications

1. Nouveaux esters contenant, à l'état chimiquement combiné, des groupes fluorocarbonés et des groupes éther oxypropylène-alkyliques, de formules

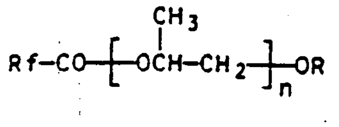

ou

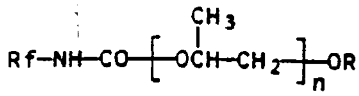

dans lesquelles

Rf représente un groupe alkyle linéaire ou ramifié en C2-C10 fluoré en totalité ou en partie, un groupe cycloalkyle en C4-C8 fluoré en totalité ou en partie, un groupe perfluorophényle ou bien un groupe perfluoralkylphényle contenant 1 à 6 atomes de carbone dans la partie perfluoralkyle,

R représente un groupe alkyle linéaire ou ramifié en C1-C4 et

n est un nombre entier allant de 2 à 70.

**2.** Nouveaux esters contenant, à l'état chimiquement combiné, des groupes fluorocarbonés et des groupes éther oxypropylène-alkyliques selon la revendication 1, caractérisés en ce que le groupe Rf contient au moins 40 % en poids de fluor.

**3.** Nouveaux esters contenant, à l'état chimiquement combiné, des groupes fluorocarbonés et des groupes oxyporpylène-alkyliques selon la revendication 1, caractérisés en ce que le groupe Rf consiste en l'un des groupes $C_3F_7$-, $C_6F_{13}$-$CH_2$, $C_6F_5$- ou $CF_3$-$C_6H_4$.

**4.** Nouveaux esters contenant, à l'état chmiquement combiné, des groupes fluorocarbonés et des groupes éther oxypropylène-alkyliques selon une des revendications 1 à 3, caractérisés en ce que le groupe polyoxypropylène consiste en 5 à 25 motifs oxypropylène.

**5.** Nouveaux esters contenant, à l'état chimiquement combiné, des groupes fluorocarbonés et des groupes éther oxypropylène-alkyliques selon une des revendications 1 à 4, caractérisés en ce que le groupe R consiste en un groupe méthyle.

**6.** Emulsions contenant des gonflants, stables à la conservation, qui contiennent :

i) au moins un hydrocarbure aliphatique et/ou cycloaliphatique en C3-C8 peu soluble ou insoluble dans ii), fluoré en totalité ou en partie, et/ou de l'hexafluorure de soufre,

ii) au moins un polyisocyanate organique et/ou un polyisocyanate modifié ou au moins un composé à haut poids moléculaire à au moins deux atomes d'hydrogène réactifs ou un mélange d'au moins un composé à haut poids moléculaire à deux atomes d'hydrogène réactifs et au moins un agent d'allongement des chaînes et/ou agent réticulant à bas poids moléculaire et

iii) au moins un ester de formule

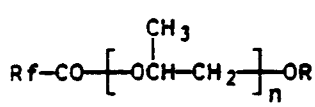

ou

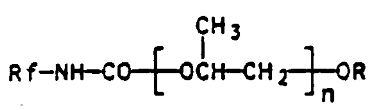 ,

dans lesquelles Rf, R et n ont les significations indiquées dans la revendication 1.

**7.** Emulsions contenant des gonflants, stables à la conservation, qui consistent en

i) 1 à 150 parties en poids, pour 100 parties en poids de (ii) d'au moins un hydrocarbure aliphatique et/ou cycloaliphatique en C3-C8 peu soluble ou insoluble dans (ii), fluoré en totalité ou en partie, et/ou de l'hexafluorure de soufre,

ii) au moins un composé à haut poids moléculaire à au moins deux atomes d'hydrogène réactifs ou un mélange d'au moins un composé à haut poids moléculaire à au moins deux atomes d'hydrogène réactifs et d'au moins un agent d'allongement des chaînes et/ou agent réticulant à bas poids moléculaire,

iii) de 0,01 à 6,0 parties en poids, pour 100 parties en poids de (ii) d'au moins un ester de formule

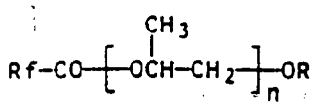

ou

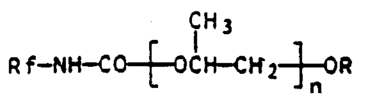

,

dans lequelles Rf, R et n ont les significations indiquées dans la revendication 1, et

iiii) 0 à 5,0 parties en poids, pour 100 parties en poids de (ii), d'au moins un polysiloxane à chaînes latérales de polyéther, qui sert de stabilisant des masses alvéolaires.

**8.** Emulsions contenant des gonflants, stables à la conservation, selon la revendication 6 ou 7, caractérisées en ce que les hydrocarbures fluorés (i) sont choisis dans le groupe formé par le perfluoropentane, le perfluorocyclopentane, le perfluorhexane, le perfluorocyclohexane, le perfluoroheptane et le perfluoroctane.

**9.** Emulsions contenant des gonflants, stables à la conservation, selon la revendication 6 ou 7, caractérisées en ce que les hydrocarbures fluorés (i) sont choisis dans le groupe des gaz liquéfiés sous une pression allant jusqu'à 100 bar : perfluoropropane, perfluorobutane et perfluorocyclobutane.

**10.** Emulsions contenant des gonflants, stables à la conservation, selon la revendication 6 ou 7, caractérisées en ce que les hydrocarbures fluorés (i) consistent en hexafluoropropane et/ou heptafluoropropane.

**11.** Emulsions contenant des gonflants, stables à la conservation, selon une des revendications 6 à 10, caractérisées en ce que les composés à haut poids moléculaire à au moins deux atomes d'hydrogène réactifs consistent en au moins un polyester-polyol de fonctionnalité 2 à 3, poids moléculaire 480 à 3000, ou au moins un polyéther-polyol de fonctionnalité 2 à 6, poids moléculaire 380 à 8000.

**12.** Emulsions contenant des gonflants, stables à la conservation, selon une des revendications 6 à 11, caractérisées en ce que l'ester (iii) est choisi parmi les composés de formules

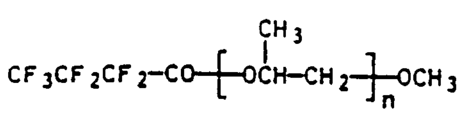

,

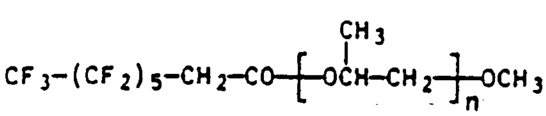

,

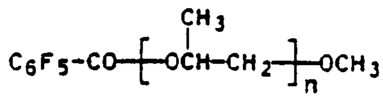

et

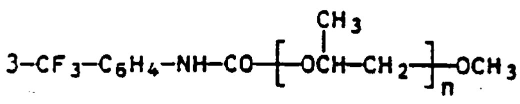,

dans lesquelles n est un nombre allant de 6 à 24.

**13.** Utilisation d'émulsions contenant des gonflants, stables à la conservation, qui contiennent

i) au moins un hydrocarbure aliphatique et/ou cycloaliphatique en C3-C8, fluoré en totalité ou en partie, peu soluble ou insoluble dans (ii) et/ou de l'hexafluorure de soufre,

ii) au moins un polyisocyanate organique et/ou un polyisocyanate organique modifié ou au moins un composé à haut poids moléculaire à au moins deux atomes d'hydrogène réactifs, ou un mélange d'au moins un composé à haut poids moléculaire à au moins deux atomes d'hydrogène réactifs et au moins un agent d'allongement des chaînes et/ou agent réticulant à bas poids moléculaire et

iii) au moins un ester de formule

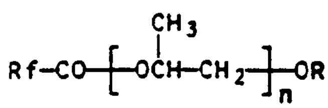

ou

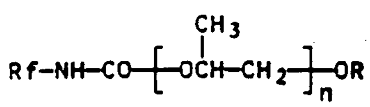,

dans lesquelles Rf, R et n ont les significations indiquées dans la revendication 1 et

iii) le cas échéant, au moins un polysiloxane à chaîne latérale de polyéther, pour la préparation de résines synthétiques cellulaires, de préférence des matières alvéolaires dures de polyuréthannes, par le procédé de polyaddition des polyisocyanates.